(19) **Europäisches Patentamt / European Patent Office / Office européen des brevets**

(11) **EP 4 567 017 A1**

(12) **EUROPEAN PATENT APPLICATION**
published in accordance with Art. 153(4) EPC

(43) Date of publication:
**11.06.2025 Bulletin 2025/24**

(21) Application number: **23849973.5**

(22) Date of filing: **26.07.2023**

(51) International Patent Classification (IPC):
**C07C 5/11** (2006.01)  **C07B 61/00** (2006.01)
**C07C 13/20** (2006.01)

(52) Cooperative Patent Classification (CPC):
(C-Sets available)
**C07C 5/11; B01J 23/60; B01J 35/393; B01J 35/45;**
**C07B 61/00; C07C 13/20;** C07C 2521/06;
C07C 2523/46; C07C 2523/60; C07C 2531/02
(Cont.)

(86) International application number:
**PCT/JP2023/027336**

(87) International publication number:
**WO 2024/029418 (08.02.2024 Gazette 2024/06)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **05.08.2022  JP 2022125754**
**05.08.2022  JP 2022125785**

(71) Applicant: **Asahi Kasei Kabushiki Kaisha**
**Tokyo 1000006 (JP)**

(72) Inventors:
• **FUKUZAWA, Akiyoshi**
**Tokyo 100-0006 (JP)**
• **ISHIHARA, Satomi**
**Tokyo 100-0006 (JP)**
• **SHIMIZU, Takayuki**
**Tokyo 100-0006 (JP)**
• **ASHIDA, Keita**
**Tokyo 100-0006 (JP)**
• **NAGANO, Yasunobu**
**Tokyo 100-0006 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **METHOD FOR PRODUCING CYCLOOLEFIN**

(57)  Provided is a method for producing a cycloolefin, comprising subjecting a monocyclic aromatic hydrocarbon to partial hydrogenation reaction with hydrogen in an aqueous zinc sulfate solution in the presence of a ruthenium catalyst, wherein
the aqueous zinc sulfate solution contains dimethylamine, and the partial hydrogenation reaction is performed with a nitrogen concentration in the aqueous zinc sulfate solution adjusted to 0.5 to 3000 mg/L or an acetic acid concentration in the aqueous zinc sulfate solution adjusted to $1 \times 10^{-3}$ to 100 mg/L.

EP 4 567 017 A1

(52) Cooperative Patent Classification (CPC): (Cont.)

C-Sets
**C07C 5/11, C07C 13/20**

**Description**

**Technical Field**

The present invention relates to a method for producing a cycloolefin.

**Background Art**

Heretofore, cycloolefins, particularly, cyclohexenes, have been widely utilized as an intermediate material for organic chemical industrial products. Various methods are known as methods for producing the cycloolefins. Among them, general methods involve partially hydrogenating a monocyclic aromatic hydrocarbon as a starting material using a ruthenium catalyst.

Many techniques or studies as to the types of catalyst components or supports, or metal salts and the like as additives to reaction systems have been proposed as methods for enhancing yields of cycloolefins. Among them, methods using a reaction system where water and zinc coexist have shown results of relatively high yields of cycloolefins.

For example, (1) a method has been proposed in which partial reduction of a monocyclic aromatic hydrocarbon with hydrogen in the presence of water is performed under neutral or acidic conditions in the presence of at least one solid basic zinc salt (except for basic zinc sulfate) using hydrogenation catalyst particles composed mainly of metal ruthenium having an average crystal diameter of 20 nm or smaller (see, for example, Patent Document 1).

(2) A method has been proposed in which when a monocyclic aromatic hydrocarbon is partially reduced with hydrogen under neutral or acidic conditions in the presence of water and at least one zinc compound, the catalyst used is a catalyst in which particles composed mainly of metal ruthenium having an average crystallite diameter of 3 to 20 nm are supported by a support (see, for example, Patent Document 2).

(3) A catalyst having, as a support, zirconia constituted by particles having an average particle size of primary particles in the range of 3 to 50 nm and an average particle size of secondary particles in the range of 0.1 to 30 $\mu$m (see, for example, Patent Document 3) has been proposed as a catalyst for producing a cycloolefin.

In the case of industrially carrying out a method for producing a cycloolefin by partially reducing a monocyclic aromatic hydrocarbon as a starting material with hydrogen using a ruthenium catalyst, it is necessary to minimize the frequency of replacement of the catalyst, which interferes with efficient production. For this reason, it is preferred that the catalyst should be able to be used for a long period.

However, the long-term use of the catalyst is known to reduce the activity of the catalyst. For example, physical change (e.g., sintering) in active site of the catalyst itself due to a reaction environment (temperature or heat of reaction), the accumulation of a poisoning substance (e.g., a sulfur compound or a dissimilar metal), and the interaction between hydrogen and ruthenium have been reported as causes of such reduction in the activity of the catalyst.

For example, (1) the case of poisoning ascribable to a sulfur compound (see, for example, Patent Document 4) and (2) the case of poisoning ascribable to a material of a reactor (see, for example, Patent Document 5) are known about the poisoning of catalysts. Further disclosed are (3) a method of removing, from a reaction system, a reported poisoning substance nickel eluted from a reactor (see, for example, Patent Document 6), (4) a method of reducing adverse effects of a chlorine ion concentration in a reaction solution (see, for example, Patent Document 7), and (5) a method using, in a reaction system, a catalyst having a chlorine content of 0.04 parts by mass or less per part by mass of ruthenium (see, for example, Patent Document 8).

For example, a method of contacting a catalyst with oxygen in a liquid phase (see, for example, Patent Document 9), a method of retaining a catalyst at a hydrogen partial pressure lower than that in hydrogenation reaction and at a temperature that does not fall below a temperature lower by 50°C than that in hydrogenation reaction (see, for example, Patent Document 10), and a method comprising the steps of: contacting a catalyst with oxygen in a liquid phase; and retaining the catalyst at a hydrogen partial pressure lower than that in hydrogenation reaction and at a temperature that does not fall below a temperature lower by 50°C than that in hydrogenation reaction (see, for example, Patent Document 11) have been proposed as methods for regenerating a ruthenium catalyst with its activity reduced due to the interaction between hydrogen and the ruthenium catalyst. Further, a method of continuously or intermittently extracting a portion of a catalyst in continuous reaction to perform regeneration treatment, and bringing the catalyst back to a reactor where partial hydrogenation reaction is performed, and an apparatus thereof (see, for example, Patent Document 12) have been proposed.

The long-term use of the catalyst is also known to change a selection rate of cycloolefin over time. For example, a method of performing reaction while changing a metal sulfate concentration in a water phase (see, for example, Patent Document 13) and a method of adding sulfuric acid to a reaction system (see, for example, Patent Document 14) have been proposed for the purpose of circumventing this event.

**List of Prior Art Documents**

**Patent Document**

Patent Document 1: Japanese Patent No. 2115581

Patent Document 2: Japanese Patent No. 2138012

Patent Document 3: Japanese Patent No. 4777891

Patent Document 4: Japanese Patent No. 1849859

Patent Document 5: Japanese Patent No. 2892233

Patent Document 6: Japanese Patent No. 5254338

Patent Document 7: Japanese Patent No. 5147053

Patent Document 8: Japanese Patent No. 3125913

Patent Document 9: Japanese Patent No. 2634828

Patent Document 10: Japanese Patent No. 2886563

Patent Document 11: Japanese Patent No. 3841226

Patent Document 12: Japanese Patent No. 4397468

Patent Document 13: Japanese Patent No. 4033980

Patent Document 14: Japanese Patent No. 4025407

**Summary of Invention**

**Problems to be Solved by Invention**

However, none of the prior literatures described above state by any means that in partial hydrogenation reaction of a monocyclic aromatic hydrocarbon with hydrogen, a nitrogen-containing component contained in an aqueous zinc sulfate solution in a reactor influences the reactivity of a catalyst.

The nitrogen-containing component is contained in the starting material monocyclic aromatic hydrocarbon or hydrogen for producing a cycloolefin and thereby comes into a reactor. Also, the nitrogen-containing component comes into a reactor via a monocyclic aromatic hydrocarbon recycled from a product separation or purification step after a partial hydrogenation reaction step.

The nitrogen-containing component derived from the starting material is contained in an original starting material for producing the monocyclic aromatic hydrocarbon or hydrogen, and probably gets mixed into a reaction system from, for example, a drug or a lubricant of equipment or the like used in a process of performing the separation or purification of the starting material. In the production of cycloolefin, the nitrogen-containing component coming into a reactor via a recycled monocyclic aromatic hydrocarbon is due to an extracting agent used in a step subsequent to a hydrogenation step or a degraded component of the extracting agent, which comes thereinto together with the recycled monocyclic aromatic hydrocarbon.

In partial hydrogenation reaction of a monocyclic aromatic hydrocarbon, generally known methods involve highly selectively enhancing a production ratio of a product of interest by adjusting a conversion rate of the reaction. **If** the conversion rate of the reaction is low, operation of recycling an unreacted form of the monocyclic aromatic hydrocarbon and bringing the monocyclic aromatic hydrocarbon back to a reactor wherein partial hydrogenation reaction is performed is carried out.

In this respect, in a separation or purification process of the partial hydrogenation reaction product from the unreacted form of the monocyclic aromatic hydrocarbon, an extraction solvent used and a degradant of the extraction solvent get mixed into the recycled monocyclic aromatic hydrocarbon and come into the reactor of partial hydrogenation reaction again.

As mentioned above, the nitrogen-containing component, when coming into the reactor for partial hydrogenation reaction, comes into contact with a catalyst at a hydrogenation reaction site and further reacts with an aqueous zinc sulfate solution, disadvantageously causing reduction in partial hydrogenation activity of the monocyclic aromatic hydrocarbon or selectivity of cycloolefin.

Meanwhile, there is no previous report about the influence of the amount of the nitrogen-containing component at this hydrogenation reaction site on hydrogenation reaction, and any method for solving this problem has not been proposed.

None of the prior literatures described above state by any means that in partial hydrogenation reaction of a monocyclic aromatic hydrocarbon with hydrogen, an acetic acid concentration in an aqueous zinc sulfate solution in a reactor influences the reactivity of the partial hydrogenation reaction.

Partial hydrogenation reaction of a monocyclic aromatic hydrocarbon requires appropriately adjusting a conversion rate of the reaction of the monocyclic aromatic hydrocarbon for highly selectively recovering a cycloolefin, the product of interest, or enhancing productivity. In this respect, an unreacted form of the monocyclic aromatic hydrocarbon is recycled to a reactor for partial hydrogenation through separation and recovery from a reaction product, in a method generally used.

Meanwhile, the reaction product obtained through partial hydrogenation reaction, also including a by-product, is similar in boiling point to the starting material monocyclic aromatic hydrocarbon. Therefore, a predetermined extracting agent is used for separation and recovery. Depending on the status of separation and recovery, the extracting agent or a degraded component of the extracting agent may get mixed into the reactor, together with the recycled monocyclic aromatic hydrocarbon.

Acetic acid is generated in a trace amount, in a separation recovery column for partial hydrogenation reaction products, as a degradation product of dimethylacetamide which exhibits excellent effectiveness for separation or purification of the starting material monocyclic aromatic hydrocarbon from the product cycloolefin and a by-product cycloparaffin. This acetic acid, if recycled, gets mixed into the recycled monocyclic aromatic hydrocarbon and then comes into the reactor for partial hydrogenation reaction.

As mentioned above, acetic acid, when coming into the reactor for partial hydrogenation reaction, acts on a catalyst at a hydrogenation reaction site, disadvantageously causing reduction in selectivity of cycloolefin.

Accordingly, an object of the present invention is to provide a method for stably producing a cycloolefin by suppressing reduction in selectivity of cycloolefin in partial hydrogenation reaction of a monocyclic aromatic hydrocarbon.

**Means for Solving Problems**

The present inventors have found that in partial hydrogenation reaction of a monocyclic aromatic hydrocarbon, reduction in activity of a catalyst and reduction in selectivity in the partial hydrogenation reaction can be markedly suppressed by controlling a nitrogen concentration in an aqueous zinc sulfate solution, whereby the problems of the conventional techniques mentioned above can be solved, leading to the completion of the present invention.

The present inventors have also found that in partial hydrogenation reaction of a monocyclic aromatic hydrocarbon, reduction in selectivity of cycloolefin in partial hydrogenation reaction can be markedly suppressed by controlling an acetic acid concentration in an aqueous zinc sulfate solution to a specific numerical range, whereby the problems of the conventional techniques mentioned above can be solved, leading to the completion of the present invention.

Specifically, the present invention is as follows.

[1] A method for producing a cycloolefin, comprising subjecting a monocyclic aromatic hydrocarbon to partial hydrogenation reaction with hydrogen in an aqueous zinc sulfate solution in the presence of a ruthenium catalyst, wherein

the aqueous zinc sulfate solution contains dimethylamine, and
the aqueous zinc sulfate solution has a nitrogen concentration of 0.5 to 3000 mg/L.

[2] The method for producing the cycloolefin according to [1], wherein
the partial hydrogenation reaction of the monocyclic aromatic hydrocarbon is performed with the nitrogen concentration in the aqueous zinc sulfate solution containing the ruthenium catalyst adjusted to 0.5 to 3000 mg/L by replacing a portion of the aqueous zinc sulfate solution with a fresh aqueous zinc sulfate solution.

[3] The method for producing the cycloolefin according to [1] or [2], wherein
**a concentration** of nitrogen contained in the monocyclic aromatic hydrocarbon is 0.003 to 35 mg/L.

[4] The method for producing the cycloolefin according to any one of [1] to [3], wherein
a ratio of a nitrogen mass to a mass of the ruthenium catalyst in the aqueous zinc sulfate solution is $5 \times 10^{-6}$ to $8 \times 10^{-2}$ times.

[5] The method for producing the cycloolefin according to any one of [1] to [4], wherein

the partial hydrogenation reaction is performed with

the nitrogen concentration in the aqueous zinc sulfate solution adjusted to 0.5 to 1500 mg/L,

a nitrogen concentration in the monocyclic aromatic hydrocarbon adjusted to 0.01 to 16 mg/L, and

a ratio of a nitrogen mass to a mass of the ruthenium catalyst in the aqueous zinc sulfate solution adjusted to $1 \times 10^{-5}$ to $5 \times 10^{-2}$ times.

[6] The method for producing the cycloolefin according to any one of [1] to [5], wherein

the aqueous zinc sulfate solution has a dimethylamine concentration of 1.7 to 9900 mg/L,

the monocyclic aromatic hydrocarbon has a dimethylamine concentration of 0.01 to 115 mg/L, and

a ratio of a dimethylamine mass to a mass of the ruthenium catalyst in the aqueous zinc sulfate solution is $2 \times 10^{-5}$ to 0.26 times.

[7] The method for producing the cycloolefin according to any one of [1] to [6], comprising:

a reaction step of subjecting the monocyclic aromatic hydrocarbon to partial hydrogenation reaction with hydrogen in the aqueous zinc sulfate solution;

an extraction step of extracting an unreacted form of the monocyclic aromatic hydrocarbon with a solvent containing a nitrogen-containing compound after the reaction step;

a water washing step of contacting the monocyclic aromatic hydrocarbon thus extracted with 0.05 to 1 times its volume of water; and

a circulation step of subjecting the monocyclic aromatic hydrocarbon thus washed with water to the reaction step, wherein

in the reaction step, a content of dimethylamine in the aqueous zinc sulfate solution is controlled to 40 to 1700 mg/L, and a ratio of a dimethylamine mass to a mass of the ruthenium catalyst is controlled to $8 \times 10^{-4}$ to $6 \times 10^{-2}$ times.

[8] A method for producing a cycloolefin, comprising subjecting a monocyclic aromatic hydrocarbon to partial hydrogenation reaction with hydrogen in an aqueous zinc sulfate solution in the presence of a ruthenium catalyst, wherein
the partial hydrogenation reaction is performed with an acetic acid concentration in the aqueous zinc sulfate solution adjusted to $1 \times 10^{-3}$ to 100 mg/L.

[9] The method for producing the cycloolefin according to [8], wherein
the partial hydrogenation reaction of the monocyclic aromatic hydrocarbon is performed with the acetic acid concentration in the aqueous zinc sulfate solution containing the ruthenium catalyst adjusted to $1 \times 10^{-3}$ to 100 mg/L by replacing a portion of the aqueous zinc sulfate solution with a fresh aqueous zinc sulfate solution.

[10] The method for producing the cycloolefin according to [8] or [9], wherein
a concentration of acetic acid contained in the monocyclic aromatic hydrocarbon is $1 \times 10^{-4}$ to 5 mg/L.

[11] The method for producing the cycloolefin according to any one of [8] to [10], wherein

a ratio of an acetic acid mass to a mass of the ruthenium catalyst in the aqueous zinc sulfate solution is

in the range of $3 \times 10^{-9}$ to $2.5 \times 10^{-3}$ times.

[12] The method for producing the cycloolefin according to any one of [8] to [11], comprising:

a reaction step of subjecting the monocyclic aromatic hydrocarbon to partial hydrogenation reaction with hydrogen in the aqueous zinc sulfate solution;

an extraction step of extracting an unreacted form of the monocyclic aromatic hydrocarbon with a solvent containing a nitrogen-containing compound after the reaction step;

a water washing step of contacting the monocyclic aromatic hydrocarbon thus extracted with 0.05 to 1 times its volume of water; and

a circulation step of subjecting the monocyclic aromatic hydrocarbon thus washed with water to the reaction step, wherein

in the reaction step, a ratio of an acetic acid mass to a mass of the ruthenium catalyst in the aqueous zinc sulfate solution is controlled to $1 \times 10^{-6}$ to $5 \times 10^{-4}$ times.

[13] The method for producing the cycloolefin according to any one of [1] to [12], wherein

the ruthenium catalyst is a zirconia-containing ruthenium catalyst,

the method comprising the step of regenerating a portion or the whole of the zirconia-containing ruthenium catalyst, wherein

the zirconia-containing ruthenium catalyst thus regenerated is recycled.

[14] The method for producing the cycloolefin according to any one of [1] to [13], wherein

the ruthenium catalyst is a zirconia-containing ruthenium catalyst, and
the zirconia-containing ruthenium catalyst has a zinc concentration of 0.5 to 3.5% by mass.

[15] The method for producing the cycloolefin according to any one of [1] to [14], wherein

the monocyclic aromatic hydrocarbon comprises

any member selected from the group consisting of benzene, toluene, and benzene substituted by an alkyl group having 1 to 4 carbon atoms.

[16] The method for producing the cycloolefin according to any one of [1] to [7], wherein
the aqueous zinc sulfate solution has an acetic acid concentration of $1 \times 10^{-3}$ mg/L to 100 mg/L.

**Advantages of Invention**

According to the present invention, a cycloolefin can be produced stably and efficiently for a long time by effectively suppressing reduction in selectivity of cycloolefin.

**Mode for Carrying Out Invention**

Hereinafter, the mode for carrying out the present invention (hereinafter, referred to the "present embodiment") will be described in detail.
The present embodiment given below is a mere illustration for explaining the present invention, and the present invention is not limited by the embodiment described below. The present invention can be carried out through appropriate changes or modifications made without departing from the spirit of the present invention.

[Method for producing cycloolefin]

The method for producing a cycloolefin according to the present embodiment (hereinafter, also simply referred to as the present embodiment) relates to a method for producing a cycloolefin, comprising subjecting a monocyclic aromatic hydrocarbon to partial hydrogenation reaction with hydrogen in an aqueous zinc sulfate solution in the presence of a ruthenium catalyst.

In the first embodiment, the aqueous zinc sulfate solution contains dimethylamine, and a nitrogen concentration in the aqueous zinc sulfate solution is set to a predetermined numerical range.

In the second embodiment, an acetic acid concentration in the aqueous zinc sulfate solution is set to a predetermined numerical range.

Hereinafter, the methods for producing a cycloolefin according to the first and second embodiments will be described.

[Method for producing cycloolefin according to first embodiment]

In the first embodiment, the aqueous zinc sulfate solution contains dimethylamine, and the aqueous zinc sulfate solution has a nitrogen concentration of 0.5 to 3000 mg/L.

According to the first embodiment, catalytic activity of partial hydrogenation reaction and selectivity of cycloolefin can be kept high, and the catalytic activity and the cycloolefin selectivity can be kept high even after catalyst regeneration. Such stabilized catalytic activity or selectivity can decrease the number of times of complicated operation such as the replacement of a catalyst or the addition of an unused catalyst in producing cycloolefin for a long time, can effectively suppress reduction in selectivity of cycloolefin, and enables cycloolefin to be produced efficiently and stably for a long time.

(Monocyclic aromatic hydrocarbon)

In the method for producing a cycloolefin according to the first embodiment, a monocyclic aromatic hydrocarbon is used as a starting material.

Examples of the monocyclic aromatic hydrocarbon include, but are not limited to, benzene, toluene, and benzene substituted by an alkyl group having 1 to 4 carbon atoms, such as xylene.

(Hydrogen pressure)

In the first embodiment, a hydrogen pressure in performing partial hydrogenation reaction with hydrogen is generally 1 to 20 MPa, preferably 2 to 7 MPa.

The hydrogen pressure is 1 MPa or higher, whereby selectivity of cycloolefin can be sufficiently secured. The hydrogen pressure is 20 MPa or lower, whereby the pressure of hydrogen or the monocyclic aromatic hydrocarbon to be supplied to a reactor can be suppressed to a practically preferred degree, a load on a facility associated with increase in pressure is reduced, and efficient operation such as supply of hydrogen and the monocyclic aromatic hydrocarbon appropriate for hydrogen consumption can be performed.

(Temperature at time of reaction)

A temperature at the time of partial hydrogenation reaction is generally 50 to 250°C, preferably 100 to 200°C.

The reaction temperature is 50°C or higher, whereby a sufficient reaction rate can be secured. The reaction temperature is 250°C or lower, whereby the growth of an average crystallite diameter (sintering) of ruthenium in the ruthenium catalyst is suppressed, and reduction in catalytic activity can be suppressed.

(Ruthenium catalyst)

In the first embodiment, the monocyclic aromatic hydrocarbon is subjected to partial hydrogenation reaction in the presence of a ruthenium catalyst.

The ruthenium catalyst is preferably a catalyst comprising metal ruthenium obtained by reducing any of various ruthenium compounds in advance.

Examples of the ruthenium compound include, but are not limited to, halide such as chloride, bromide, and iodide, nitrate, sulfate, hydroxide, various complexes containing ruthenium, for example, ruthenium-carbonyl complexes, ruthenium-acetyl acetate complexes, ruthenocene complexes, ruthenium-ammine complexes, and ruthenium-hydride complexes, and compounds derived from these complexes.

One of these ruthenium compounds may be used singly, or two or more thereof can be used as a mixture.

Examples of methods for reducing these ruthenium compounds include catalytic reduction methods with hydrogen or carbon monoxide, and chemical reduction methods with formalin, sodium borohydride, potassium borohydride, or hydrazine.

Among these reduction methods, preferred methods are catalytic reduction with hydrogen and a chemical reduction method with sodium borohydride.

In the case of catalytic reduction with hydrogen, reductive activation is performed under a condition of usually 50 to 450°C, preferably 100 to 400°C, more preferably 100 to 250°C.

The reduction temperature is 50°C or higher, whereby reduction is achieved at a practically sufficient rate. The reduction temperature is 450°C or lower, whereby the aggregation of ruthenium can be suppressed, and adverse effects on activity or selectivity can be prevented. This reduction may be performed in a gas phase or performed in a liquid phase, and liquid-phase reduction is preferred. In the case of chemical reduction with sodium borohydride, the reduction temperature is preferably a temperature equal to or lower than 100°C, usually preferably 10°C to 80°C.

The state of the ruthenium catalyst to be added at the time of partial hydrogenation reaction may be a ruthenium compound state containing no metal ruthenium. In this case, the ruthenium compound is preferably a compound such as hydroxide containing no chlorine ion.

In the case of adding a ruthenium catalyst containing no metal ruthenium at the time of partial hydrogenation reaction, it is preferred to use a ruthenium hydroxide-supported form obtained by allowing the ruthenium compound mentioned above to be supported by a support and treating the resultant with an alkali such as sodium hydroxide, or to use a mixture of ruthenium hydroxide and a dispersant obtained by adding an alkali such as sodium hydroxide to a mixture containing the dispersant and the ruthenium compound described above.

The ruthenium compound that may be used can be a ruthenium-based compound obtained by adding another metal or metal compound, for example, zinc, chromium, molybdenum, tungsten, manganese, cobalt, nickel, iron, copper, gold, platinum, boron, lanthanum, or cerium, or a compound of such a metal before or after reduction of the ruthenium compound.

In the case of using such another metal or metal compound, its atomic ratio to a ruthenium atom is usually selected in the range of 0.001 to 20. The another metal or metal compound is preferably a zinc or a zinc compound. This zinc or zinc compound is preferably added before or during reduction of the ruthenium compound, and the amount of zinc or the zinc compound added is preferably 0.001 to 2 times (in terms of zinc) the mass of ruthenium. Zinc is more preferably used in 0.005 to 1 times the mass of ruthenium from the viewpoint of catalytic activity and selectivity of cycloolefin.

Examples of methods for preparing such a ruthenium-based catalyst containing another metal or metal compound include (1) a method of allowing the ruthenium compound and another metal or metal compound to be supported by a support, followed by reduction treatment, (2) a method of adding an alkali such as sodium hydroxide to a solution containing the ruthenium compound and another metal or metal compound, and reducing the resulting precipitate as an insoluble salt of the ruthenium compound and the metal or the like together, (3) a method of allowing, if necessary, the insoluble ruthenium compound to be supported by a support, and reducing this ruthenium compound in a liquid phase containing another metal compound or the like, and (4) a method of performing reduction treatment in a state where both the ruthenium compound and another metal compound are dissolved in a liquid phase.

In the method for producing a cycloolefin according to the first embodiment, a catalyst supported by a support may be used.

Examples of the support include, but are not particularly limited to, oxide, complex oxide, hydroxide, and poorly water-soluble metal salts of a metal such as magnesium, aluminum, silicon, calcium, titanium, vanadium, chromium, manganese, cobalt, iron, nickel, copper, zinc, zirconium, hafnium, tungsten, or boron, and compounds and mixtures of two or more of these metals combined chemically or physically.

Among them, the support is preferably zirconium oxide (zirconia) or zirconium hydroxide. Particularly, zirconium oxide (zirconia) is preferred because of its excellent physical stability such as a specific surface area under reaction conditions. An average particle size of the zirconium oxide is preferably 0.05 to 30 $\mu$m, more preferably 0.05 to 10 $\mu$m. Its specific surface area is preferably 20 to 200 $m^2$/g, and its average pore size is preferably 1 to 50 nm.

Examples of methods for allowing ruthenium to be supported by the support include, but are not particularly limited to, adsorption methods, ion exchange methods, dipping methods, coprecipitation methods, and drying methods.

The amount of the support used is not particularly limited and is usually 1 to 1000 times the mass of ruthenium. Particularly, in using zirconium oxide (zirconia) as the support, it is preferred to use zirconium oxide in 1 to 100 times, further preferably 2 to 20 times, the mass of ruthenium.

In the method for producing a cycloolefin according to the first embodiment, a zirconia-containing ruthenium catalyst is preferably used in the partial hydrogenation reaction of the monocyclic aromatic hydrocarbon. In the case of using a zirconia-containing ruthenium catalyst, the support is zirconia, or without the use of the support, zirconium is used as a dispersant, as mentioned later, and oxidized into zirconia, which in turn forms the catalyst together with ruthenium.

In the case of using ruthenium directly without being supported by the support, it is preferred to use a dispersant such as oxide, complex oxide, hydroxide, and poorly water-soluble metal salts of a metal such as magnesium, aluminum, silicon, calcium, titanium, vanadium, chromium, manganese, cobalt, iron, nickel, copper, zinc, zirconium, hafnium, tungsten, barium, or boron, or a compound or a mixture of two or more of these metals combined chemically or physically.

An average crystallite diameter of metal ruthenium of the ruthenium catalyst used in the first embodiment is preferably 20 nm or smaller. The average crystallite diameter of 20 nm or smaller is preferred because sufficiently many active sites exist owing to a preferred surface area of the catalyst so that catalytic activity is improved. The lower limit value of this average crystallite diameter is theoretically a value larger than a crystal unit and is realistically 1 nm or larger.

(Aqueous zinc sulfate solution)

In the method for producing a cycloolefin according to the first embodiment, the monocyclic aromatic hydrocarbon is subjected to partial hydrogenation reaction in an aqueous zinc sulfate solution. Specifically, use of water is a key requirement. The amount of water differs depending on a reaction format. The amount of water is 0.5 to 20 times the mass of the starting material monocyclic aromatic hydrocarbon. Within this range, selectivity of cycloolefin can be kept high without increasing the size of a reactor. The amount of water is more preferably 1 to 10 times the mass of the monocyclic aromatic hydrocarbon used. In any case, it is required that the reaction system should contain water in an amount that attains a phase-separated state of an organic liquid phase (oil phase) composed mainly of the starting material and a product, and water, i.e., a two-liquid-phase state, an oil phase and a water phase. In this context, the phrase "composed mainly of" refers to a component that exhibits the largest proportion in terms of the number of moles among components constituting a liquid phase.

The reaction system according to the first embodiment is performed in the aqueous zinc sulfate solution. Zinc sulfate needs to exist in an at least partially or wholly dissolved state in the water phase.

<Zinc sulfate and metal sulfate>

In the production method of the first embodiment, the reaction system may contain another metal sulfate in addition to zinc sulfate.

Examples of the another metal sulfate include sulfate of iron, nickel, cadmium, gallium, indium, magnesium, aluminum, chromium, manganese, cobalt, or copper. Two or more thereof may be used in combination, or a double salt containing such metal sulfate may be used.

A content of zinc sulfate in the aqueous zinc sulfate solution is preferably $1 \times 10^{-3}$ to 2.0 mol/L in terms of a concentration in the water phase from the viewpoint of enhancing catalytic activity and selectivity of cycloolefin. The content is more preferably 0.1 to 1.0 mol/L, further preferably 0.4 to 0.9 mol/L.

For a concentration of metal sulfate other than zinc sulfate, it is preferred to appropriately set the concentration depending on the type of a metal component.

<Metal salt>

In the method for producing a cycloolefin according to the first embodiment, the reaction system may contain a metal salt described below, as in conventional methods.

Examples of the metal salt include nitrate, oxide, hydroxide, acetate, and phosphate of metals in group 1 of the periodic table such as lithium, sodium, and potassium, metals in group 2 thereof such as magnesium and calcium (the group number is based on the IUPAC Nomenclature of Inorganic Chemistry, Revised (1989)), or metals such as zinc, manganese, cobalt, copper, cadmium, lead, arsenic, iron, gallium, germanium, vanadium, chromium, silver, gold, platinum, nickel, palladium, barium, aluminum, and boron. Two or more thereof may be used as a chemical and/or physical mixture. However, metal chloride is not preferred because a chlorine ion concentration in the water phase has adverse effects on long-term performance of the catalyst. The metal salt except for chloride or the metal oxide is preferably a zinc compound such as zinc hydroxide or zinc oxide, particularly preferably a double salt containing zinc hydroxide, for example, a double salt represented by the general formula $(ZnSO_4)_m \cdot (Zn(OH)_2)_n$ wherein m:n = 1:0.01 to 1:100.

An amount of the above-mentioned metal salt used is not particularly limited as long as the water phase is kept acidic or neutral. Usually, the amount is $1 \times 10^{-5}$ to $1 \times 10^5$ times the mass of ruthenium used. The metal salt may exist at any location in the reaction system, and its form of existence does not necessarily require that the whole amount is dissolved in the water phase.

<pH of water phase of aqueous zinc sulfate solution>

pH in the water phase of the aqueous zinc sulfate solution for use in the method for producing a cycloolefin according to the first embodiment is preferably acidic or neutral pH of 7.0 or lower from the viewpoint of enhancing catalytic activity and selectivity of cycloolefin.

<Chlorine ion concentration>

In the method for producing a cycloolefin according to the first embodiment, a chlorine ion dissolved in water of the reaction system might have adverse effects on long-term performance of the catalyst, as mentioned above. For this reason, a concentration of the chlorine ion is preferably 300 mg/L or lower, more preferably 200 mg/L or lower, further preferably 100 mg/L or lower.

The chlorine ion concentration refers to the total concentration of all chlorine atoms and chlorine existing in the form of ions, including all of combined residual chlorine existing in the form of chloramine through reaction of free residual chlorine existing by keeping equilibrium of chlorine $Cl_2$, hypochlorous acid HClO, and a hypochlorite ion $ClO^-$, with an ammonia ion present in water, and chloride ions existing in the form of $Cl^-$.

The chlorine ion concentration is 300 mg/L or lower, whereby in the first embodiment, reduction in activity and reduction in cycloolefin selectivity can be effectively suppressed when the ruthenium catalyst is used for a long time.

The reason is not certain why a chlorine ion dissolved in water reduces reaction activity of the ruthenium catalyst and cycloolefin selectivity, is presumably the following.

In the presence of a chlorine ion, ruthenium is dissolved in water so that re-reduction occurs. This promotes sintering of ruthenium, causing reduction in activity of the catalyst. At the same time therewith, the interaction of ruthenium with its coexisting metal or metal compound is changed. This metal or metal compound has an important role in cycloolefin selectivity, presumably also reducing cycloolefin selectivity.

In the first embodiment, the concentration of the chlorine ion dissolved in the water phase containing the ruthenium catalyst can be controlled to 300 mg/L or lower by controlling the chlorine ion concentration of each constituent present in the water phase.

Specifically, the concentration of the chlorine ion dissolved in the water phase containing the ruthenium catalyst can be controlled to 300 mg/L or lower by controlling, to a give value or lower, the concentrations of chlorine ions respectively contained in water, the catalyst, a dispersant, metal sulfate, a metal salt, the monocyclic aromatic hydrocarbon, and hydrogen to be supplied to the reaction system.

In the first embodiment, an effective approach involves supplying the monocyclic aromatic hydrocarbon and hydrogen to a reaction site as well as freshly supplying water in an amount corresponding to a content of water discharged from the reaction site together with a reaction solution. A chlorine ion content of the water to be freshly supplied is preferably 20 mg/L or lower, more preferably 10 mg/L or lower, further preferably 5 mg/L or lower.

Examples of methods for controlling the chlorine ion content in the water to be freshly supplied to the range mentioned above include methods such as treatment with an ion exchange resin and distillation purification.

<Electric conductivity of water to be supplied to reaction site>

An electric conductivity of the water to be supplied to the reaction site is preferably 0.5 $\mu$S/cm or less, more preferably 0.3 $\mu$S/cm or less, in terms of an electric conductivity generally used from the viewpoint of keeping catalytic activity and selectivity of cycloolefin high.

<Nitrogen concentration in aqueous zinc sulfate solution>

In the method for producing a cycloolefin according to the first embodiment, a nitrogen concentration in the aqueous zinc sulfate solution is set to the range of 0.5 to 3000 mg/L.

The nitrogen concentration in the aqueous zinc sulfate solution means a concentration obtained by determining a content of a nitrogen-containing component as a content of a nitrogen atom. The nitrogen concentration in the aqueous zinc sulfate solution can be measured by a method described in Examples mentioned later.

Examples of the nitrogen-containing component that gets mixed into the aqueous zinc sulfate solution include impurities of the starting material monocyclic aromatic hydrocarbon or hydrogen for producing a cycloolefin, products from a process of separating or purifying the starting material, and drugs or lubricating oils of equipment or the like used in this process as well as extracting agents used in the step of separating or purifying products after a partial hydrogenation reaction step, and degradation components of the extracting agents, which get mixed into a recycled monocyclic aromatic hydrocarbon.

Examples of the nitrogen-containing component that gets mixed into the aqueous zinc sulfate solution by accompanying the starting material monocyclic aromatic hydrocarbon or hydrogen include nitrogen-containing components such as monoethanolamine, ammonia, pyrrole, pyridine, and quinoline. Examples of the extracting agent and the degradation component of the extracting agent, which come into a reactor via a recycled monocyclic aromatic hydrocarbon include dimethylacetamide and dimethylamine.

The aqueous zinc sulfate solution for use in the method for producing a cycloolefin according to the first embodiment contains dimethylamine. Further examples of the nitrogen-containing component dissolved in the aqueous zinc sulfate solution include monoethanolamine, ammonia, and dimethylacetamide.

In the first embodiment, diligent studies have been made on the influence of a nitrogen concentration of a reaction system for partial hydrogenation of a monocyclic aromatic hydrocarbon on the reactivity of the partial hydrogenation reaction of the monocyclic aromatic hydrocarbon. As a result, a nitrogen-containing component has been found to react with an aqueous zinc sulfate solution, further change physical properties of a catalyst, and influence the reactivity. Accordingly, a nitrogen concentration in the aqueous zinc sulfate solution, a nitrogen concentration in the monocyclic aromatic hydrocarbon, and a ratio of a nitrogen mass to a catalyst mass have been controlled.

In the first embodiment, the total nitrogen concentration in the aqueous zinc sulfate solution is set to the range of 0.5 to 3000 mg/L in light of the influence of the nitrogen-containing component on the partial hydrogenation reaction of the monocyclic aromatic hydrocarbon.

The nitrogen concentration of 3000 mg/L or lower in the aqueous zinc sulfate solution can suppress reduction in reaction activity of the ruthenium catalyst and can prevent reduction in selectivity.

The concentration of nitrogen dissolved in the aqueous zinc sulfate solution influences reduction in reaction activity of the ruthenium catalyst or selectivity due to the following cause.

Most nitrogen-containing components coming into a reaction site exhibit alkalinity and are responsible for salting out zinc in the aqueous zinc sulfate solution. The salted-out zinc acts on the ruthenium catalyst and thereby reduces reaction activity by modifying a reactive point of the ruthenium catalyst. The nitrogen-containing component is considered to also act as a poisoning substance on the ruthenium catalyst so as to reduce selectivity. Stable production of cycloolefin requires operation of suppressing reduction in reaction activity of the ruthenium catalyst and adopts a method of removing the salted-out zinc responsible for reduction in catalytic activity. Specifically, a method of suppressing reduction in reaction activity by dissolving the salted-out zinc by the addition of sulfuric acid to a reaction site of the aqueous zinc sulfate solution can be carried out. However, the added sulfuric acid acts on the ruthenium catalyst so as to restore the reduced activity, and on the other hand, influences reduction in selectivity such as the alteration of the original ruthenium catalyst itself.

In view of the description above, the nitrogen concentration in the aqueous zinc sulfate solution is controlled to 3000 mg/L or lower, preferably 1500 mg/L or lower, more preferably 1000 mg/L or lower, further preferably 650 mg/L or lower.

A method of keeping a concentration low as to a nitrogen-containing component in a starting material for use in producing a cycloolefin, including a recycled monocyclic aromatic hydrocarbon, is effective as a method for controlling the concentration of nitrogen dissolved in the aqueous zinc sulfate solution to 3000 mg/L or lower.

For the nitrogen-containing component coming into a reactor via a starting material for the monocyclic aromatic hydrocarbon or hydrogen, it is preferred to sufficiently decrease a concentration of the nitrogen-containing component in a starting material purification step. For the nitrogen-containing component coming into a reactor via a recycled monocyclic aromatic hydrocarbon, it is also preferred to decrease a concentration of the nitrogen-containing component in the monocyclic aromatic hydrocarbon by distillation purification in the separation and recovery of the monocyclic aromatic hydrocarbon from an extracting agent used as the nitrogen-containing component, or to add the step of removing the nitrogen-containing component.

However, the decrease of the nitrogen concentration in the aqueous zinc sulfate solution has limitations. In reality, it is preferred to control the nitrogen concentration in the aqueous zinc sulfate solution to 0.5 mg/L or higher in light of a large scale of a removal facility for the nitrogen-containing component, a large energy load in the operation of the removal facility, and large economic burden. The nitrogen concentration is 15 mg/L or higher, whereby energy load in the operation of the removal facility and economic burden can be reduced.

(Replacement of a portion of aqueous zinc sulfate solution)

The nitrogen concentration in the aqueous zinc sulfate solution is attributed to a nitrogen-containing component coming thereinto from a starting material including a recycled monocyclic aromatic hydrocarbon, whereas all of incoming nitrogen-containing components do not accumulate in the aqueous zinc sulfate solution and a portion thereof is extracted to the outside of the reactor, together with a reaction product. In short, the nitrogen concentration in the aqueous zinc sulfate solution can be decreased by reducing the amount of the nitrogen-containing component coming into a reaction site.

On the other hand, for reducing the amount of the nitrogen-containing component coming thereinto from a starting material including a recycled monocyclic aromatic hydrocarbon, an approach is required such as the elevation of a load in the step of purifying each starting material or the addition of the step of removing the nitrogen-containing component. Furthermore, an elevated nitrogen concentration in the aqueous zinc sulfate solution at a reaction site requires a lot of time for lowering the nitrogen concentration in the aqueous zinc sulfate solution even if the nitrogen-containing component is gradually extracted to the outside of the reactor, together with a reaction product. Hence, studies have been made on a method for lowering the nitrogen concentration in the aqueous zinc sulfate solution at a reaction site. As a result, the replacement of a portion of the aqueous zinc sulfate solution with a fresh aqueous zinc sulfate solution having a low nitrogen concentration has been found effective.

The nitrogen concentration once elevated in the aqueous zinc sulfate solution is reduced by replacement operation with a fresh aqueous zinc sulfate solution so that reactivity reduced due to the influence of the nitrogen concentration is restored. In short, if the nitrogen concentration in the aqueous zinc sulfate solution temporarily falls outside the proper range of 0.5 to 3000 mg/L, the nitrogen concentration can be brought back to the proper range by replacing a portion of the aqueous zinc sulfate solution with a fresh aqueous zinc sulfate solution, as mentioned above.

(Nitrogen concentration in monocyclic aromatic hydrocarbon)

In the method for producing a cycloolefin according to the first embodiment, a concentration of nitrogen contained in the monocyclic aromatic hydrocarbon is preferably in the range of 0.003 to 35 mg/L.

In this case, the monocyclic aromatic hydrocarbon includes both of the monocyclic aromatic hydrocarbon initially used as a starting material in a production process, and a recycled monocyclic aromatic hydrocarbon.

The nitrogen-containing component coming into a reaction site from the starting material monocyclic aromatic hydrocarbon includes a component derived from the original starting material, a nitrogen-containing component generated in a separation or purification process, and a nitrogen-containing component that gets mixed thereinto by originating from a drug or a lubricating oil of equipment or the like used.

The nitrogen-containing component coming into a reactor via a monocyclic aromatic hydrocarbon recycled from the step of separating or purifying products after a partial hydrogenation reaction step includes an extracting agent used in a step subsequent to the hydrogenation step, and a degraded component of the extracting agent.

Since the nitrogen-containing component is impurities of the monocyclic aromatic hydrocarbon, it is necessary for removing the impurities or decreasing the amount of the impurities to adjust a facility for separation or purification or operating conditions. The removal of the nitrogen-containing component having a distinctive boiling point by distillation purification requires the design of a distillation column and the application of operating conditions for achieving a target nitrogen concentration in the monocyclic aromatic hydrocarbon.

The reduction or removal of the nitrogen-containing component using an adsorbent or a facility that can extract and remove the nitrogen-containing component from the monocyclic aromatic hydrocarbon is required for a method for reducing or removing impurities, other than distillation purification.

Examples of methods for reducing or removing the nitrogen-containing component using an adsorbent include methods using an adsorption column packed with active alumina, silica-alumina, smectite, or zeolite as the adsorbent.

In the case of reducing or removing a nitrogen-containing component soluble in water, a method using a water washing column is effective such as a method of dipping the monocyclic aromatic hydrocarbon in water.

When the nitrogen-containing component is generated in the course of starting material purification or recycling treatment, for example, a device to minimize the amount of the nitrogen-containing component getting mixed into the monocyclic aromatic hydrocarbon by suppressing a causative factor thereof is required. For example, a method of lowering a temperature at a generation location of the nitrogen-containing component is effective when the nitrogen-containing component is a pyrolyzed substance. An approach such as a method of preventing water from getting mixed thereinto is effective when the nitrogen-containing component is a hydrolysis product.

The nitrogen concentration in a total monocyclic aromatic hydrocarbon coming into a reaction site of the aqueous zinc sulfate solution is 35 mg/L or lower, whereby the nitrogen concentration in the aqueous zinc sulfate solution is easily controlled to 3000 mg/L or lower, and reduction in reactivity of partial hydrogenation reaction can be prevented.

In short, the nitrogen concentration in the total monocyclic aromatic hydrocarbon is preferably 35 mg/L or lower, more preferably 16 mg/L or lower, further preferably 10 mg/L or lower.

A nitrogen concentration in the total monocyclic aromatic hydrocarbon lower than 0.003 mg/L increases facility cost for reducing or removing the nitrogen-containing component or increases an operational load or a load on analysis and management in low-concentration management of a trace nitrogen-containing component. Therefore, the lower limit value of the nitrogen concentration in the total monocyclic aromatic hydrocarbon is preferably 0.003 mg/L or higher, more preferably 0.01 mg/L or higher from the viewpoint of a reduced load on management.

The nitrogen concentration in the monocyclic aromatic hydrocarbon can be measured by a method described in Examples mentioned later.

The concentration of nitrogen contained in the monocyclic aromatic hydrocarbon can be controlled to the numerical range mentioned above by carrying out a nitrogen component removal step such as the purification of the starting material monocyclic aromatic hydrocarbon or the distillation purification of the recycled monocyclic aromatic hydrocarbon.

(Ratio of nitrogen mass of nitrogen-containing component to ruthenium catalyst mass)

In the method for producing a cycloolefin according to the first embodiment, a ratio of a nitrogen mass of the nitrogen-containing component to a mass of the ruthenium catalyst in the aqueous zinc sulfate solution is preferably set to the range of $5 \times 10^{-6}$ to $8 \times 10^{-2}$ times. The ratio is more preferably $1 \times 10^{-5}$ to $5 \times 10^{-2}$ times, further preferably $1.5 \times 10^{-5}$ to $3 \times 10^{-2}$ times.

The ratio of a nitrogen mass to a mass of the ruthenium catalyst in the aqueous zinc sulfate solution can be calculated by extracting the aqueous zinc sulfate solution containing the ruthenium catalyst in a completely mixed state from a hydrogenation reaction site, performing filtration, water washing, and drying, and measuring a solid ruthenium catalyst while measuring a nitrogen concentration in a filtrate by a method described in Examples mentioned later to determine masses of both the components.

The ratio of a nitrogen mass to a mass of the ruthenium catalyst can be controlled to the numerical range described above by treatment to reduce the nitrogen concentration in the aqueous zinc sulfate solution or the adjustment of an amount of the ruthenium catalyst added.

In the first embodiment, a ruthenium catalyst is used as a catalyst for producing a cycloolefin through the partial hydrogenation reaction of the monocyclic aromatic hydrocarbon. Ruthenium in the aqueous zinc sulfate solution plays a role in adsorbing the monocyclic aromatic hydrocarbon and hydrogen and desorbing the resulting cycloolefin. In such a role, when the nitrogen-containing component comes into the aqueous zinc sulfate solution, zinc in the aqueous zinc sulfate solution is solidified as zinc hydroxide or a double salt of zinc sulfate and zinc hydroxide. This zinc hydroxide or double salt of zinc sulfate and zinc hydroxide inhibits partial hydrogenation reaction in such a way as to conceal a reactive point of ruthenium, and has influence to reduce reaction activity.

Meanwhile, stable production of cycloolefin requires, for example, an approach of reducing zinc responsible for reduction in activity by adding sulfuric acid into the aqueous zinc sulfate solution. The reduced activity is restored by the addition of sulfuric acid to a reaction site, whereas selectivity of cycloolefin is reduced because zinc in the catalyst, which is effective for the adsorption of the monocyclic aromatic hydrocarbon or hydrogen to ruthenium or the cycloolefin-desorbing action at the stage of the original catalyst design, is also dissolved by the addition of sulfuric acid. Hence, it is necessary to reduce the ratio of a nitrogen mass to a mass of the ruthenium catalyst without having influence to inhibit the reaction of the ruthenium catalyst.

From the viewpoint mentioned above, the amount of the nitrogen-containing component is preferably adjusted such that the ratio of a nitrogen mass of the nitrogen-containing component to a mass of the ruthenium catalyst in the aqueous zinc sulfate solution is $8 \times 10^{-2}$ times or less, more preferably $5 \times 10^{-2}$ times or less, further preferably $3 \times 10^{-2}$ times or less.

A ratio of a nitrogen mass of the nitrogen-containing component to a mass of the ruthenium catalyst in the aqueous zinc sulfate solution kept at less than $5 \times 10^{-6}$ times increases facility cost for reducing or removing the nitrogen-containing component in the purification or separation of the starting material including a recycled one, or increases an operational load or a load on analysis and management in low-concentration management of a trace nitrogen-containing component.

In addition, the nitrogen-containing component present in a tiny amount in the aqueous zinc sulfate solution without having influence on reduction in activity of the ruthenium catalyst and requiring the management of sulfuric acid addition might contribute to the stabilization of selectivity of cycloolefin through the effect of salting out zinc in a trace amount. Therefore, the ratio of a nitrogen mass to a mass of the ruthenium catalyst in the aqueous zinc sulfate solution is preferably not less than $5 \times 10^{-6}$ times, more preferably not less than $1 \times 10^{-5}$ times, further preferably not less than $1.5 \times 10^{-5}$ times.

In the first embodiment, the cycloolefin is preferably produced by subjecting the monocyclic aromatic hydrocarbon to partial hydrogenation reaction, wherein, as mentioned above, the nitrogen concentration in the aqueous zinc sulfate solution is set to 0.5 to 1500 mg/L, the nitrogen concentration in the monocyclic aromatic hydrocarbon is set to 0.01 to 16 mg/L, and the ratio of a nitrogen mass to a mass of the ruthenium catalyst in the aqueous zinc sulfate solution is set to $1 \times 10^{-5}$ to $5 \times 10^{-2}$ times.

This can keep high catalytic activity of the partial hydrogenation reaction of the monocyclic aromatic hydrocarbon and selectivity of cycloolefin, can stabilize ruthenium catalyst performance, decrease the number of times of complicated operation such as the replacement of the ruthenium catalyst or the addition of the ruthenium catalyst in producing cycloolefin for a long time, effectively suppresses reduction in selectivity of cycloolefin, and enables cycloolefin to be produced stably and efficiently for a long time.

(Nitrogen-containing component)

The nitrogen-containing component that exhibits a nitrogen concentration in the aqueous zinc sulfate solution according to the present embodiment includes a substance that is contained in the starting material monocyclic aromatic hydrocarbon or hydrogen for producing a cycloolefin and thereby comes into a reaction system as well as a substance that comes into a reaction system via a recycled monocyclic aromatic hydrocarbon.

Examples of such substances include components such as dimethylamine, dimethylacetamide, monoethanolamine, ammonia, pyrrole, pyridine, and quinoline.

According to investigation and studies by the present inventors, dimethylamine occupies a large proportion of substances contained in the aqueous zinc sulfate solution, among these nitrogen-containing components.

In the method for producing a cycloolefin according to the first embodiment, the aqueous zinc sulfate solution contains dimethylamine.

The dimethylamine is a degradation product of dimethylacetamide used as an extraction solvent in the step of separating or purifying products after a partial hydrogenation reaction step.

Dimethylamine generated by hydrolysis with trace water introduced into the product separation or purification step, pyrolysis, or the like gets mixed into a recycled monocyclic aromatic hydrocarbon and comes into the aqueous zinc sulfate solution in the hydrogenation reaction system.

The hydrolysis of dimethylacetamide produces acetic acid in addition to dimethylamine. This acetic acid serves as a catalyst that promotes the degradation of dimethylacetamide, and influences increase in dimethylamine level. Dimethylacetamide coming into the hydrogenation reaction system may be hydrolyzed in the aqueous zinc sulfate solution to form dimethylamine. Due to such influence, dimethylamine occupies a large proportion of nitrogen-containing components contained in the aqueous zinc sulfate solution.

In light of the description above, in the first embodiment, it is important to control a concentration of dimethylamine in the aqueous zinc sulfate solution.

(Concentration of dimethylamine in aqueous zinc sulfate solution)

In the method for producing a cycloolefin according to the first embodiment, a concentration of dimethylamine in the aqueous zinc sulfate solution to be supplemented with the ruthenium catalyst is preferably in the range of 1.7 to 9900 mg/L, more preferably in the range of 1.7 to 4950 mg/L, still more preferably in the range of 1.7 to 3300 mg/L, even more preferably in the range of 40 to 1700 mg/L, further preferably in the range of 80 to 1000 mg/L.

The concentration of dimethylamine in the aqueous zinc sulfate solution is 1.7 mg/L or higher, whereby an effect of reducing a load on an apparatus or an operational load as well as an effect of stably maintaining ruthenium catalyst performance can be obtained. The concentration is 9900 mg/L or lower, whereby an effect of suppressing reduction in ruthenium catalytic activity or cycloolefin selectivity can be obtained.

The concentration of dimethylamine in the aqueous zinc sulfate solution can be measured by a method described in Examples mentioned later.

The concentration of dimethylamine in the aqueous zinc sulfate solution can be controlled to the numerical range described above, for example, by adjusting the performance of distillation separation of dimethylamine coming into a reactor via a recycled monocyclic aromatic hydrocarbon, adjusting conditions of a facility for removing a nitrogen-containing component in a monocyclic aromatic hydrocarbon before entrance into a reactor, and adjusting conditions of the step of extracting and separating a source of dimethylamine as well as extracting an aqueous zinc sulfate solution containing dimethylamine to the outside of a reaction system and replenishing a reactor with a fresh aqueous zinc sulfate solution.

(Dimethylamine concentration in monocyclic aromatic hydrocarbon)

A concentration of dimethylamine in a total monocyclic aromatic hydrocarbon coming into a reaction site of the aqueous zinc sulfate solution, including a recycled monocyclic aromatic hydrocarbon, is preferably in the range of 0.01 to 115 mg/L, more preferably in the range of 0.04 to 52 mg/L, further preferably in the range of 0.04 to 33 mg/L.

The concentration of dimethylamine in the monocyclic aromatic hydrocarbon is 0.01 mg/L or higher, whereby an effect of reducing a load on an apparatus or an operational load as well as an effect of stably maintaining ruthenium catalyst performance can be obtained. The concentration is 115 mg/L or lower, whereby an effect of suppressing elevation in dimethylamine concentration in the aqueous zinc sulfate solution can be obtained.

The concentration of dimethylamine in the monocyclic aromatic hydrocarbon can be measured by a method described in Examples mentioned later.

The concentration of dimethylamine in the monocyclic aromatic hydrocarbon can be controlled to the numerical range described above, for example, by adjusting conditions of separation or purification of the monocyclic aromatic hydrocarbon, adjusting conditions of a dimethylamine removal facility, and adjusting conditions of the step of extracting and separating a source of dimethylamine.

(Ratio of mass of dimethylamine to ruthenium catalyst mass)

A ratio of a dimethylamine mass to a mass of the ruthenium catalyst in the aqueous zinc sulfate solution is preferably in the range of $2 \times 10^{-5}$ to 0.26 times, more preferably in the range of $4 \times 10^{-5}$ to 0.16 times, still more preferably in the range of $5 \times 10^{-5}$ to 0.1 times, even more preferably in the range of $8 \times 10^{-4}$ to $6 \times 10^{-2}$ times, further preferably in the range of $1 \times 10^{-3}$ to $4 \times 10^{-2}$ times.

The ratio of a dimethylamine mass to a mass of the ruthenium catalyst is $2 \times 10^{-5}$ times or more, whereby an effect of reducing a load on an apparatus or an operational load as well as an effect of stably maintaining ruthenium catalyst performance can be obtained. The ratio is 0.26 times or less, whereby an effect of stabilizing ruthenium catalytic activity and cycloolefin selectivity can be obtained.

The ratio of a dimethylamine mass to a mass of the ruthenium catalyst can be measured by a method described in Examples mentioned later.

The ratio of a dimethylamine mass to a mass of the ruthenium catalyst can be controlled to the numerical range described above by treatment to reduce the dimethylamine concentration in the aqueous zinc sulfate solution or the adjustment of an amount of the ruthenium catalyst added.

(Acetic acid concentration in aqueous zinc sulfate solution)

In the method for producing a cycloolefin according to the first embodiment, an acetic acid concentration in the aqueous zinc sulfate solution is preferably in the range of $1 \times 10^{-3}$ to 100 mg/L.

The acetic acid comes into a reactor via a monocyclic aromatic hydrocarbon recycled from a monocyclic aromatic hydrocarbon purification step for recycling after a partial hydrogenation reaction step and subsequent separation of a product.

A reaction solution after partial hydrogenation reaction contains a partially hydrogenated cycloolefin, a completely hydrogenated cycloalkane, and an unreacted form of the monocyclic aromatic hydrocarbon. In order to separate this reaction solution, dimethylacetamide is used as a known extracting agent. When this dimethylacetamide is used as an extracting agent, acetic acid is produced as a degradation product. If the acetic acid cannot be sufficiently separated in the step of purifying a recycled monocyclic aromatic hydrocarbon, the acetic acid gets mixed into a partial hydrogenation reaction site together with the recycled monocyclic aromatic hydrocarbon.

The acetic acid concentration in the aqueous zinc sulfate solution serving as a reaction site is 100 mg/L or lower, whereby reduction in reaction selectivity of the catalyst can be suppressed. The acetic acid concentration in the aqueous zinc sulfate solution is preferably 75 mg/L or lower, more preferably 50 mg/L or lower.

Possible influence of acetic acid on reaction selectivity of the catalyst is adverse effects on the action of adsorbing or desorbing the starting material monocyclic aromatic hydrocarbon or cycloolefin to or from the catalyst. At a reaction site, the monocyclic aromatic hydrocarbon dissolved in the water phase, i.e., the aqueous zinc sulfate solution, is converted to cycloolefin through partial hydrogenation on the catalyst and eliminated from the catalyst. In the presence of acetic acid, the acetic acid modifies catalyst surface and accelerates the progression of oil-phase reaction of the monocyclic aromatic hydrocarbon, leading to increase in cycloalkane level. In addition, possible influence in the water phase is, for example, the unlikelihood of elimination of a reaction product from the catalyst surface in the process from cycloolefin to cycloalkane.

The acetic acid concentration in the aqueous zinc sulfate solution can be measured by a method described in Examples mentioned later.

The acetic acid concentration in the aqueous zinc sulfate solution can be measured by ion chromatography (IC).

Examples of methods for controlling the acetic acid concentration in the aqueous zinc sulfate solution to 100 mg/L or lower include methods of keeping a concentration low as to acetic acid contained in a recycled monocyclic aromatic hydrocarbon.

In the separation of the recycled monocyclic aromatic hydrocarbon from an extracting agent, the acetic acid concentration in the recycled monocyclic aromatic hydrocarbon is effectively lowered by properly setting distillation purification conditions. Since acetic acid is a degradation product of dimethylacetamide used as an extracting agent, an effective approach involves preventing water which promotes degradation from participating in an extraction and separation step involving dimethylacetamide, or involves preventing a temperature from becoming too high in a separation step.

However, the decrease of the acetic acid concentration in the aqueous zinc sulfate solution has practical limitations. It is preferred to control the acetic acid concentration in the aqueous zinc sulfate solution to $1 \times 10^{-3}$ mg/L or higher in light of a load on a purification or separation facility or a load on operating conditions. It is easier to control the acetic acid concentration to $1 \times 10^{-2}$ mg/L or higher.

In the first embodiment, acetic acid in the aqueous zinc sulfate solution comes thereinto from a starting material including a recycled monocyclic aromatic hydrocarbon, whereas all of incoming acetic acids do not accumulate in the aqueous zinc sulfate solution and a portion thereof is extracted to the outside of the reactor, together with a reaction product. In short, the acetic acid concentration in the aqueous zinc sulfate solution can be decreased by reducing the amount of the acetic acid coming into a reaction site.

On the other hand, for reducing the amount of the acetic acid coming thereinto from a starting material including a recycled monocyclic aromatic hydrocarbon, an approach of elevating a load in the step of separating or purifying the recycled monocyclic aromatic hydrocarbon, or suppressing the degradation of the extracting agent dimethylacetamide is required. Furthermore, it takes a lot of time to reduce the amount of acetic acid by gradually extracting the acetic acid that has once came into the aqueous zinc sulfate solution, together with a reaction product. Hence, a method of replacing a portion of the aqueous zinc sulfate solution with a fresh aqueous zinc sulfate solution containing no acetic acid is effective as a method for lowering the acetic acid concentration in the aqueous zinc sulfate solution at a reaction site.

The acetic acid concentration once elevated in the aqueous zinc sulfate solution is reduced by replacement operation with a fresh aqueous zinc sulfate solution so that reactivity reduced due to the influence of the acetic acid is restored. In short, if the acetic acid concentration in the aqueous zinc sulfate solution temporarily falls outside the proper range of $1 \times 10^{-3}$ to 100 mg/L, the acetic acid concentration can be brought back to the proper range by the replacement operation.

(Concentration of acetic acid contained in monocyclic aromatic hydrocarbon)

In the method for producing a cycloolefin according to the first embodiment, a concentration of acetic acid contained in the monocyclic aromatic hydrocarbon is preferably set to the range of $1 \times 10^{-4}$ to 5 mg/L.

In this case, the monocyclic aromatic hydrocarbon includes both of the monocyclic aromatic hydrocarbon initially used in a production process, and a recycled monocyclic aromatic hydrocarbon.

The level of acetic acid in the monocyclic aromatic hydrocarbon can be reduced and can be controlled to the numerical range described above, for example, by suppressing its generation in an extraction and separation step, performing separation or purification to remove or reduce the generated acetic acid as impurities of the monocyclic aromatic hydrocarbon, reducing or removing the acetic acid using an adsorbent material, or treating the monocyclic aromatic hydrocarbon by water washing.

The acetic acid concentration in the monocyclic aromatic hydrocarbon is specified to 5 mg/L or lower, whereby a rate at which the acetic acid concentration in the aqueous zinc sulfate solution is gradually elevated is suppressed, and reduction in performance of partial hydrogenation reaction can be prevented.

In short, the acetic acid concentration in the monocyclic aromatic hydrocarbon is preferably 5 mg/L or lower, more preferably 3 mg/L or lower, further preferably 1.5 mg/L or lower.

On the other hand, an acetic acid concentration in the monocyclic aromatic hydrocarbon lower than $1 \times 10^{-4}$ mg/L increases an operational load or a load on analysis and management in the reduction or removal of or low-concentration management of acetic acid. Therefore, the lower limit value is preferably $1 \times 10^{-4}$ mg/L or higher, more preferably $1 \times 10^{-3}$ mg/L or higher, whereby an operational load or a load on analysis in separation or purification can be reduced.

The concentration of acetic acid contained in the monocyclic aromatic hydrocarbon is determined by adding water to an aromatic hydrocarbon sample to be measured, followed by concentration and extraction, and measuring the extracted water by ion chromatography analysis. In addition, a sample having a concentration of 1 mg/L or higher can be analyzed by use of gas chromatography (GC).

(Ratio of acetic acid mass to ruthenium catalyst mass)

In the method for producing a cycloolefin according to the first embodiment, a ratio of an acetic acid mass to a mass of the ruthenium catalyst in the aqueous zinc sulfate solution is preferably set to the range of $3 \times 10^{-9}$ to $2.5 \times 10^{-3}$ times. The ratio is more preferably $1 \times 10^{-8}$ to $2 \times 10^{-3}$ times, further preferably $1 \times 10^{-7}$ to $1.5 \times 10^{-3}$ times, still further preferably $1 \times 10^{-6}$ to $5 \times 10^{-4}$ times, even further preferably $3 \times 10^{-6}$ to $5 \times 10^{-4}$ times. The ratio of an acetic acid mass to a mass of the ruthenium catalyst in the aqueous zinc sulfate solution can be calculated as a ratio of a total solid catalyst concentration (mg/L) in a reaction solution to the amount of acetic acid (mg/L) in the aqueous zinc sulfate solution.

In the first embodiment, a ruthenium catalyst is used as a catalyst for producing a cycloolefin through the partial hydrogenation reaction of the monocyclic aromatic hydrocarbon.

Ruthenium in the aqueous zinc sulfate solution plays a role in adsorbing the monocyclic aromatic hydrocarbon and hydrogen and desorbing the resulting cycloolefin. In such a role, acetic acid coming into the aqueous zinc sulfate solution inhibits the reactivity of the ruthenium catalyst. Hence, the amount of acetic acid is preferably in a range having no influence to inhibit the reaction of the ruthenium catalyst. It is preferred to control the acetic acid concentration in the aqueous zinc sulfate solution for acetic acid present at a reaction site, the concentration of acetic acid contained in a recycled monocyclic aromatic hydrocarbon, and the acetic acid concentration per catalyst. If the acetic acid concentration in the aqueous zinc sulfate solution or the acetic acid concentration per catalyst is elevated to above the preferred range, the reactivity of the ruthenium catalyst can be restored by bringing the concentration back to the preferred range by the replacement of the aqueous zinc sulfate solution at a reaction site.

(Preferred form of method for producing cycloolefin according to first embodiment)

The method for producing a cycloolefin according to the first embodiment preferably comprises:

a reaction step of subjecting the monocyclic aromatic hydrocarbon to partial hydrogenation reaction with hydrogen in the aqueous zinc sulfate solution;

an extraction step of extracting an unreacted form of the monocyclic aromatic hydrocarbon with a solvent containing a nitrogen-containing compound after the reaction step;

a water washing step of contacting the monocyclic aromatic hydrocarbon thus extracted with 0.05 to 1 times its volume of water; and

a circulation step of subjecting the monocyclic aromatic hydrocarbon thus washed with water to the reaction step, wherein

in the reaction step, a content of dimethylamine in the aqueous zinc sulfate solution is controlled to 40 to 1700 mg/L, and a ratio of a dimethylamine mass to a mass of the ruthenium catalyst is controlled to $8 \times 10^{-4}$ to $6 \times 10^{-2}$ times.

As a result, catalytic activity of partial hydrogenation reaction and selectivity of cycloolefin can be kept high, and the catalytic activity and the cycloolefin selectivity can be kept high even after catalyst regeneration. Such stabilized catalytic activity can decrease the number of times of complicated operation such as the replacement of a catalyst or the addition of an unused catalyst in producing cycloolefin for a long time, can effectively suppress reduction in selectivity of cycloolefin, and enables cycloolefin to be produced efficiently and stably for a long time.

The content of dimethylamine in the aqueous zinc sulfate solution can be controlled to the numerical range described above, for example, by adjusting the performance of distillation separation of dimethylamine coming into a reactor via a recycled monocyclic aromatic hydrocarbon, adjusting conditions of a facility for removing a nitrogen-containing component in a monocyclic aromatic hydrocarbon before entrance into a reactor, and adjusting conditions of the step of extracting and separating a source of dimethylamine as well as extracting an aqueous zinc sulfate solution containing dimethylamine to the outside of a reaction system and replenishing a reactor with a fresh aqueous zinc sulfate solution.

The water to be contacted in the water washing step has preferably 0.05 to 0.8 times, more preferably 0.08 to 0.6 times, the volume of the monocyclic aromatic hydrocarbon.

The ratio of a dimethylamine mass to a mass of the ruthenium catalyst can be controlled to the numerical range described above by treatment to reduce the dimethylamine level in the aqueous zinc sulfate solution or the adjustment of the amount of the ruthenium catalyst added.

[Method for producing cycloolefin according to second embodiment]

The method for producing a cycloolefin according to the second embodiment (hereinafter, also simply referred to as the present embodiment) relates to a method for producing a cycloolefin, comprising subjecting a monocyclic aromatic hydrocarbon to partial hydrogenation reaction with hydrogen in an aqueous zinc sulfate solution in the presence of a ruthenium catalyst.

In the second embodiment, the partial hydrogenation reaction is performed with an acetic acid concentration in the aqueous zinc sulfate solution adjusted to $1 \times 10^{-3}$ to 100 mg/L.

According to the second embodiment, catalytic activity of partial hydrogenation reaction and selectivity of cycloolefin can be kept high, and the catalytic activity and the cycloolefin selectivity can be kept high even after catalyst regeneration. Such stabilized catalytic activity can decrease the number of times of complicated operation such as the replacement of a catalyst or the addition of an unused catalyst in producing cycloolefin for a long time, can effectively suppress reduction in selectivity of cycloolefin, and enables cycloolefin to be produced efficiently and stably for a long time.

(Monocyclic aromatic hydrocarbon)

In the method for producing a cycloolefin according to the second embodiment, a monocyclic aromatic hydrocarbon is used as a starting material.

Examples of the monocyclic aromatic hydrocarbon include, but are not limited to, benzene, toluene, and benzene substituted by an alkyl group having 1 to 4 carbon atoms, such as xylene. The same monocyclic aromatic hydrocarbon as in the first embodiment mentioned above can be used.

(Hydrogen pressure)

In the second embodiment, a hydrogen pressure in performing partial hydrogenation reaction with hydrogen is generally 1 to 20 MPa, preferably 2 to 7 MPa, from the same viewpoint as in the first embodiment mentioned above.

(Temperature at time of reaction)

A temperature at the time of partial hydrogenation reaction is generally 50 to 250°C, preferably 100 to 200°C, from the same viewpoint as in the first embodiment mentioned above.

(Ruthenium catalyst)

In the second embodiment, the monocyclic aromatic hydrocarbon is subjected to partial hydrogenation reaction in the presence of a ruthenium catalyst.

The ruthenium catalyst is preferably a catalyst comprising metal ruthenium obtained by reducing any of various ruthenium compounds in advance. The same ruthenium catalyst as in the first embodiment mentioned above can be used.

(Aqueous zinc sulfate solution)

In the method for producing a cycloolefin according to the second embodiment, the monocyclic aromatic hydrocarbon is subjected to partial hydrogenation reaction in an aqueous zinc sulfate solution. The aqueous zinc sulfate solution can employ the same material as that for the aqueous zinc sulfate solution used in the first embodiment mentioned above. In the second embodiment, an acetic acid concentration in the aqueous zinc sulfate solution is specified to a predetermined numerical range. The acetic acid concentration will be mentioned later.

<Acetic acid concentration in aqueous zinc sulfate solution>

In the method for producing a cycloolefin according to the second embodiment, an acetic acid concentration in the aqueous zinc sulfate solution is set to the range of $1 \times 10^{-3}$ to 100 mg/L.

The acetic acid comes into a reactor via the starting material monocyclic aromatic hydrocarbon. Particularly, the acetic acid comes into a reactor via a monocyclic aromatic hydrocarbon recycled from a monocyclic aromatic hydrocarbon purification step for recycling after a partial hydrogenation reaction step and subsequent separation of a product.

A reaction solution after partial hydrogenation reaction contains a partially hydrogenated cycloolefin, a completely hydrogenated cycloalkane, and an unreacted form of the monocyclic aromatic hydrocarbon. In order to separate this reaction solution, dimethylacetamide is used as a known extracting agent. When this dimethylacetamide is used as an extracting agent, acetic acid is produced as a degradation product. If the acetic acid cannot be sufficiently separated in the step of purifying a recycled monocyclic aromatic hydrocarbon, the acetic acid gets mixed into a partial hydrogenation reaction site together with the recycled monocyclic aromatic hydrocarbon.

The acetic acid concentration in the aqueous zinc sulfate solution serving as a reaction site is 100 mg/L or lower, whereby reduction in reaction selectivity of the catalyst can be suppressed. The acetic acid concentration in the aqueous zinc sulfate solution is preferably 75 mg/L or lower, more preferably 50 mg/L or lower.

Possible influence of acetic acid on reaction selectivity of the catalyst is adverse effects on the action of adsorbing or desorbing the starting material monocyclic aromatic hydrocarbon or cycloolefin to or from the catalyst. At a hydrogenation reaction site, the monocyclic aromatic hydrocarbon dissolved in the water phase, i.e., the aqueous zinc sulfate solution, is converted to cycloolefin through partial hydrogenation on the catalyst and eliminated from the catalyst. In the presence of acetic acid, the acetic acid modifies catalyst surface and accelerates the progression of oil-phase reaction of the monocyclic aromatic hydrocarbon, leading to increase in cycloalkane level. In addition, possible influence in the water phase is, for example, the unlikelihood of elimination of a reaction product from the catalyst surface in the process from cycloolefin to cycloalkane.

The acetic acid concentration in the aqueous zinc sulfate solution can be measured by a method described in Examples mentioned later.

The acetic acid concentration in the aqueous zinc sulfate solution can be measured by ion chromatography (IC).

Examples of methods for controlling the acetic acid concentration in the aqueous zinc sulfate solution to 100 mg/L or lower include methods of keeping a concentration low as to acetic acid contained in a recycled monocyclic aromatic hydrocarbon.

In the step of separating the recycled monocyclic aromatic hydrocarbon from an extracting agent, the acetic acid concentration in the recycled monocyclic aromatic hydrocarbon is effectively lowered by properly setting distillation purification conditions. The level of acetic acid generated by distillation purification is effectively reduced by the application of a method of discharging the acetic acid together with other impurities to the outside of the system from the extraction and separation step using a purification column for an extracting agent dimethylacetamide, or a method of lowering the level of acetic acid in a recycled monocyclic aromatic hydrocarbon using a water washing column. Since acetic acid is a degradation product of dimethylacetamide used as an extracting agent, an effective device involves preventing water which promotes degradation from participating in an extraction and separation step involving dimethylacetamide, or involves preventing a temperature from becoming too high in a separation step.

However, the decrease of the acetic acid concentration in the aqueous zinc sulfate solution has practical limitations. It is preferred to control the acetic acid concentration in the aqueous zinc sulfate solution to $1 \times 10^{-3}$ mg/L or higher in light of a load on a purification or separation facility or a load on operating conditions. It is easier to control the acetic acid concentration to $1 \times 10^{-2}$ mg/L or higher.

(Replacement of a portion of aqueous zinc sulfate solution)

In the second embodiment, acetic acid in the aqueous zinc sulfate solution comes thereinto from a starting material including a recycled monocyclic aromatic hydrocarbon, whereas all of incoming acetic acids do not accumulate in the aqueous zinc sulfate solution and a portion thereof is extracted to the outside of the reactor, together with a reaction product. In short, the acetic acid concentration in the aqueous zinc sulfate solution can be decreased by reducing the amount of the acetic acid coming into a reaction site.

On the other hand, for reducing the amount of the acetic acid coming thereinto from a starting material including a recycled monocyclic aromatic hydrocarbon, an approach of elevating a load in the step of separating or purifying the recycled monocyclic aromatic hydrocarbon, or suppressing the degradation of the extracting agent dimethylacetamide is required. Furthermore, it takes a lot of time to gradually reduce the amount of acetic acid that has once came into the aqueous zinc sulfate solution, together with a reaction product. Hence, a method of replacing a portion of the aqueous zinc sulfate solution with a fresh aqueous zinc sulfate solution, i.e., an aqueous zinc sulfate solution containing no acetic acid, is effective as a method for lowering the acetic acid concentration in the aqueous zinc sulfate solution at a reaction site.

The acetic acid concentration once elevated in the aqueous zinc sulfate solution is reduced by replacement operation with a fresh aqueous zinc sulfate solution so that reactivity reduced due to the influence of the acetic acid is restored. In short, if the acetic acid concentration in the aqueous zinc sulfate solution temporarily falls outside the proper range of $1 \times 10^{-3}$ to 100 mg/L, the acetic acid concentration can be brought back to the proper range by the replacement operation.

(Concentration of acetic acid contained in monocyclic aromatic hydrocarbon)

In the method for producing a cycloolefin according to the second embodiment, a concentration of acetic acid contained in the monocyclic aromatic hydrocarbon is preferably set to the range of $1 \times 10^{-4}$ to 5 mg/L.

In this case, the monocyclic aromatic hydrocarbon includes both of the monocyclic aromatic hydrocarbon initially used in a production process, and a recycled monocyclic aromatic hydrocarbon.

The level of acetic acid in the monocyclic aromatic hydrocarbon can be reduced and can be controlled to the numerical range described above, for example, by suppressing its generation in the step of extracting and separating a recycled monocyclic aromatic hydrocarbon, performing separation or purification to remove or reduce the generated acetic acid as impurities of the monocyclic aromatic hydrocarbon, reducing or removing the acetic acid using an adsorbent material, or treating the monocyclic aromatic hydrocarbon by water washing.

The acetic acid concentration in the monocyclic aromatic hydrocarbon is specified to 5 mg/L or lower, whereby the acetic acid concentration in the aqueous zinc sulfate solution containing the ruthenium catalyst can be prevented from being gradually elevated, the acetic acid concentration in the aqueous zinc sulfate solution can be adjusted to 100 mg/L or lower, and reduction in performance of partial hydrogenation reaction can be prevented.

In short, the acetic acid concentration in the monocyclic aromatic hydrocarbon is preferably 5 mg/L or lower, more preferably 3 mg/L or lower, further preferably 1.5 mg/L or lower.

On the other hand, an acetic acid concentration in the monocyclic aromatic hydrocarbon lower than $1 \times 10^{-4}$ mg/L increases an operational load or a load on analysis and management in the reduction or removal of or low-concentration management of acetic acid. Therefore, the lower limit value is preferably $1 \times 10^{-4}$ mg/L or higher, more preferably $1 \times 10^{-3}$ mg/L or higher, whereby an operational load or a load on analysis in separation or purification can be further reduced.

The acetic acid concentration in the monocyclic aromatic hydrocarbon can be measured by a method described in Examples mentioned later.

(Ratio of acetic acid mass to ruthenium catalyst mass)

In the method for producing a cycloolefin according to the second embodiment, a ruthenium catalyst is used as a catalyst for producing a cycloolefin through the partial hydrogenation reaction of the monocyclic aromatic hydrocarbon.

In the second embodiment, a ratio of an acetic acid mass to a mass of the ruthenium catalyst in the aqueous zinc sulfate solution is preferably set to the range of $3 \times 10^{-9}$ to $2.5 \times 10^{-3}$ times. The ratio is more preferably $1 \times 10^{-8}$ to $2 \times 10^{-3}$ times, further preferably $1 \times 10^{-7}$ to $1.5 \times 10^{-3}$ times, still further preferably $1 \times 10^{-6}$ to $5 \times 10^{-4}$ times.

The ratio of an acetic acid mass to a mass of the ruthenium catalyst can be calculated by a method described in Examples mentioned later.

The partial hydrogenation reaction of the monocyclic aromatic hydrocarbon is performed through the ruthenium catalyst which adsorbs the monocyclic aromatic hydrocarbon and hydrogen and eliminates the resulting cycloolefin from the catalyst, in the aqueous zinc sulfate solution. In such reaction, acetic acid coming into the aqueous zinc sulfate solution inhibits the adsorption and desorption characteristics of the ruthenium catalyst and reduces reactivity.

Hence, the acetic acid concentration in the aqueous zinc sulfate solution is preferably in a range having no influence to inhibit the reaction of the ruthenium catalyst. It is necessary to control the acetic acid concentration in the aqueous zinc sulfate solution serving as a reaction site, the concentration of acetic acid contained in a recycled monocyclic aromatic hydrocarbon, and the ratio of an acetic acid mass to a mass of the ruthenium catalyst.

If the acetic acid concentration in the aqueous zinc sulfate solution or the ratio of an acetic acid mass to a mass of the ruthenium catalyst falls above the preferred range, the reactivity of the ruthenium catalyst can be restored by bringing the concentration or the ratio back to the preferred range by the replacement of the aqueous zinc sulfate solution at a reaction site.

(Preferred form of method for producing cycloolefin according to second embodiment)

The method for producing a cycloolefin according to the second embodiment preferably comprises:

a reaction step of subjecting the monocyclic aromatic hydrocarbon to partial hydrogenation reaction with hydrogen in the aqueous zinc sulfate solution;

an extraction step of extracting an unreacted form of the monocyclic aromatic hydrocarbon with a solvent containing a nitrogen-containing compound after the reaction step;

a water washing step of contacting the monocyclic aromatic hydrocarbon thus extracted with 0.05 to 1 times its volume of water; and

a circulation step of subjecting the monocyclic aromatic hydrocarbon thus washed with water to the reaction step, wherein

in the reaction step, a ratio of an acetic acid mass to a mass of the ruthenium catalyst in the aqueous zinc sulfate solution is controlled to $1 \times 10^{-6}$ to $5 \times 10^{-4}$ times.

As a result, catalytic activity of partial hydrogenation reaction and selectivity of cycloolefin can be kept high, and the catalytic activity and the cycloolefin selectivity can be kept high even after catalyst regeneration. Such stabilized catalytic activity can decrease the number of times of complicated operation such as the replacement of a catalyst or the addition of an unused catalyst in producing cycloolefin for a long time, can effectively suppress reduction in selectivity of cycloolefin, and enables cycloolefin to be produced efficiently and stably for a long time.

The water to be contacted in the water washing step has preferably 0.05 to 0.8 times, more preferably 0.08 to 0.6 times, the volume of the monocyclic aromatic hydrocarbon.

The ratio of an acetic acid mass to a mass of the ruthenium catalyst in the aqueous zinc sulfate solution can be controlled to the numerical range described above by the replacement of the aqueous zinc sulfate solution at a reaction site.

[Regeneration of ruthenium catalyst]

In the methods for producing a cycloolefin according to the first and second embodiments, a portion or the whole of the ruthenium catalyst can be regenerated and recycled.

The regeneration and recycling of a portion or the whole of the ruthenium catalyst can suppress reduction in activity of the catalyst and cycloolefin selectivity.

The regeneration of a portion or the whole of the ruthenium catalyst produces an effect of removing a poisoning component on reactive surface of the catalyst and produces an effect of regenerating the altered activity of the catalyst after use.

The ruthenium catalyst to be regenerated is particularly preferably a zirconia-containing ruthenium catalyst from the viewpoint of enhancing regeneration efficiency and extending the duration of catalyst life.

In the methods for producing a cycloolefin according to the first and second embodiments, examples of substances whose abundance needs to be controlled in the aqueous zinc sulfate solution include nitrogen-containing components, particularly, dimethylamine, and acetic acid. These substances are considered to participate in the generation of substances reducing reaction performance or to poison or alter reactive surface of the catalyst.

Particularly, when the partial hydrogenation reaction of the monocyclic aromatic hydrocarbon is continuously performed, a substance poisoning or altering the ruthenium catalyst present in the aqueous zinc sulfate solution is strongly bonded to the surface of the ruthenium catalyst and thereby tends to become difficult to eliminate or remove, as the time passes. The aggregation of a ruthenium catalyst component also proceeds gradually. The poisoning or altering substance, if present, is incorporated into the aggregation of the ruthenium catalyst component and tends to become more difficult to eliminate or remove from the catalyst surface.

The catalyst regeneration treatment can eliminate or remove the catalyst-poisoning or -altering substance strongly bonded to the catalyst surface as mentioned above, can suppress the aggregation of the catalyst, and can suppress reduction in activity of the catalyst or selectivity of cycloolefin by redispersing the aggregated catalyst component and thereby detaching the catalyst-poisoning or - altering substance from the catalyst surface.

An amount of the ruthenium catalyst regenerated may be the whole or may be a portion and can be appropriately selected depending on a production line. A method for regenerating the catalyst can be carried out in a batch manner or continuously.

The regeneration of the whole of the catalyst has the following advantage.

Specifically, in the case of performing batch reaction, the regeneration treatment in the whole amount is preferred from the viewpoint that the activity of the catalyst thus regenerated and cycloolefin selectivity are easily controlled by grasping regeneration conditions and the relationship between the activity of the regenerated catalyst and the selectivity through a basic experiment.

On the other hand, the regeneration of a portion of the catalyst has the following advantage.

When a portion of the catalyst is regenerated, the catalyst treated by regeneration and a catalyst without regeneration treatment are recycled in admixture. The state of hydrogen and a metal ion adsorbed in optimum amounts onto ruthenium is preferred for the activity of the catalyst and cycloolefin selectivity. The admixture of the catalyst treated by regeneration, which involves acute change on ruthenium, and a catalyst without regeneration treatment can create the state of hydrogen and a metal ion adsorbed relatively mildly onto ruthenium, and is preferred from the viewpoint that activity and cycloolefin selectivity can be retained in good balance for long-term use of the catalyst.

In the case of producing a cycloolefin through batch reaction, the term "a portion" is preferably 5% by mass to 80% by mass, more preferably 10% by mass to 60% by mass, of the catalyst used in the reaction. In reality, the amount of the catalyst regenerated in continuous reaction is changed depending on the degree of reduction in catalyst performance per time. For example, it is preferred for regeneration to treat 5% by mass to 80% by mass, more preferably 10% by mass to 60% by mass, of the whole catalyst in 24 hours.

A method for extracting a portion or the whole of the catalyst from continuous reaction, performing regeneration treatment, and bringing the catalyst back to a reactor where partial hydrogenation reaction is performed is not particularly limited. For example, in the case of continuous reaction, the method may involve temporarily discontinuing the continuous reaction, removing an oil phase, and after regeneration treatment of the whole liquid phase containing the catalyst, resuming partial hydrogenation reaction. Alternatively, the liquid phase containing the catalyst may be partially extracted without discontinuing the continuous reaction, and the reactor for partial hydrogenation reaction may be refilled with the catalyst while regeneration treatment is performed.

Examples of apparatuses for continuously extracting the liquid phase containing the catalyst and performing regeneration treatment and refilling, and methods for operating the apparatuses include methods and apparatuses consisting of a partial hydrogenation reactor, a jacketed condenser, and a jacketed activation treatment machine, disclosed in Japanese Patent No. 4397468.

Known methods can be used as methods for regenerating the ruthenium catalyst.

Examples thereof include (1) a method of contacting the catalyst with oxygen in a liquid phase, and (2) a method of retaining the catalyst at a hydrogen partial pressure lower than that in hydrogenation reaction and at a temperature that does not fall below a temperature lower by 50°C than that at the time of partial hydrogenation reaction.

In the method (1), which is a method of contacting the catalyst with oxygen in a liquid phase, the state of the liquid phase can be a state where the ruthenium catalyst is dispersed in a slurry form in an appropriate liquid. Although the liquid can be used in a small amount, at least the surface of the catalyst needs to be covered with the liquid. The liquid used can be any liquid having no adverse effects on the ruthenium catalyst or its support, and is preferably water.

In the case of contacting the catalyst with oxygen in a liquid phase, an oxygen gas or a gas containing molecular oxygen, such as air, can be used as an oxygen source, or a compound that generates nascent oxygen, such as hydrogen peroxide, can be used. The oxygen gas may be used directly and is preferably used after being diluted with an appropriate inert gas, for convenient operation.

An oxygen concentration of the liquid phase is usually $1 \times 10^{-7}$ to 1 NmL/mL, preferably $1 \times 10^{-5}$ to 0.1 NmL/mL, based on a standard-state oxygen gas. The oxygen concentration within this range renders a treatment time relatively short and can prevent ruthenium on the catalyst surface from being irreversibly changed due to acute oxidation.

Oxygen can be supplied directly to the liquid phase in a slurry state. The most preferred method for supplying oxygen involves dispersing the ruthenium catalyst in water and supplying a gas containing oxygen to this dispersion. This method is preferred because of its convenient operation.

Operation of restoring the activity of the ruthenium catalyst may be carried out under any condition of reduced pressure, ordinary pressure, or pressurization.

Pressurization may be performed in order to elevate the oxygen concentration of the liquid phase.

A temperature of the operation of contacting the catalyst with oxygen can be in the range of 0 to 300°C and is preferably 30 to 200°C, more preferably 50 to 150°C.

An operation time can be appropriately determined depending on the degree of reduction in activity of the catalyst to be treated or a target degree of activity restoration and is usually several minutes to several days.

The method (2), which is a regeneration method of retaining the catalyst at a hydrogen partial pressure lower than that in hydrogenation reaction and at a temperature that does not fall below a temperature lower by 50°C than that at the time of partial hydrogenation reaction, may be carried out in a gas phase or a liquid phase.

The hydrogen partial pressure can be lower than that in hydrogenation reaction. However, a small difference between both the hydrogen partial pressures may require a long time for activity restoration. Therefore, the hydrogen partial pressure is preferably set to 1/2 or less of the pressure in partial hydrogenation reaction, more preferably a pressure of zero or nearly zero.

The temperature of the operation of retaining the catalyst is set to a temperature that does not fall below a temperature lower by 50°C, preferably by 40°C, more preferably by 30°C, than that at the time of partial hydrogenation reaction. The operation temperature may exceed the temperature at the time of partial hydrogenation reaction. However, too high a temperature might cause irreversible change in active site of the catalyst. Therefore, it is preferred to select the upper limit of the operation temperature suitable for the characteristics of the catalyst.

As for fine particles of the ruthenium catalyst for use in the first or second embodiment, it is preferred to retain the catalyst at a temperature of not higher than 250°C, more preferably a temperature of not higher than 200°C, from the viewpoint of preventing physical change in the catalyst. On the other hand, a temperature that falls below a temperature lower by 50°C than that at the time of hydrogenation reaction may require treatment for a markedly long time for activity restoration. A retention time can be appropriately selected depending on the degree of reduction in activity of the catalyst to be treated or a target degree of activity restoration and is usually several minutes to several days.

In the case of combining the two methods mentioned above, i.e., the methods (1) and (2) for regenerating the ruthenium catalyst, either of the methods may be carried out first. Preferably, the method (1) of contacting the catalyst with oxygen in a liquid phase is performed first. For performing such operation of activity restoration, it is preferred to separate and remove partial hydrogenation reaction-derived organic matter that coexists with the catalyst, in advance from the ruthenium catalyst. In this context, the organic matter that coexist with the catalyst means, for example, the starting material monocyclic aromatic hydrocarbon, a reaction product, a by-product, or impurities.

The ruthenium catalyst that has restored its activity is appropriately subjected to washing or drying operation to prepare a preferred form, which is then recycled to the partial hydrogenation reaction of the monocyclic aromatic hydrocarbon.

The activity of the catalyst and selectivity of cycloolefin found to have reduction in performance, and selectivity can be restored to some extent by extracting a portion or the whole of the ruthenium catalyst and carrying out only regeneration treatment. However, this approach may not always be sufficient. Reduction in activity of the ruthenium catalyst and cycloolefin selectivity can be effectively suppressed by maintaining a concentration of a nitrogen-containing component, particularly, dimethylamine, dissolved in the aqueous zinc sulfate solution, or an acetic acid concentration in a proper range, in addition to the regeneration treatment of the ruthenium catalyst. This can also enhance the performance of regeneration of the ruthenium catalyst and can therefore maintain high catalytic activity and selectivity of cycloolefin even after regeneration treatment.

The mechanism under which deterioration in the catalyst is suppressed by maintaining a concentration of a nitrogen-containing component, particularly, dimethylamine, or an acetic acid concentration in a proper range, in addition to the regeneration treatment of the ruthenium catalyst is considered to render the nitrogen-containing component or acetic acid difficult to eliminate or remove from the catalyst surface in the regeneration treatment of the catalyst if the concentration of each component is high so as to exceed the proper range. Furthermore, a poisoning substance temporarily eliminated from the catalyst surface by the regeneration treatment of the catalyst may be re-adsorbed to the catalyst surface, presumably reducing reaction performance again.

Specifically, even in the case of extracting a portion or the whole of the ruthenium catalyst and performing regeneration treatment, it is preferred to adjust the concentration of the nitrogen-containing component dissolved in the aqueous zinc sulfate solution to the range of 0.5 to 3000 mg/L, it is preferred to adjust the concentration of dimethylamine to the range of 1.7 to 9900 mg/L, and it is preferred to adjust the acetic acid concentration to the range of $1 \times 10^{-3}$ to 100 mg/L.

The concentration of each component maintained in the proper range facilitates eliminating or removing a poisoning substance or an altering substance adsorbed on the ruthenium catalyst from the catalyst surface, and produces an effect of being able to suppress reduction in activity of the catalyst and reduction in selectivity not only for a short period but for a long period.

[Zinc concentration in zirconia-containing ruthenium catalyst]

In the first and second embodiments, a concentration of zinc in the ruthenium catalyst is preferably maintained in the range of 0.5 to 3.5% by mass. The concentration is more preferably 0.5 to 2.5% by mass, further preferably 0.5 to 1.8% by mass.

The zinc concentration in the ruthenium catalyst can be measured by a method described in Examples mentioned later.

When the concentration of zinc falls within the numerical range described above, a zirconia-containing ruthenium catalyst is particularly preferred from the viewpoint of improvement in stability of the zinc concentration at the time of reaction and the stabilization of reactivity of the catalyst.

Nitrogen-containing components, dimethylacetamide, and dimethylamine coming into a reaction site of the aqueous zinc sulfate solution exhibit alkalinity. These substances, when coming into the aqueous zinc sulfate solution, are responsible for salting out zinc. The salted-out zinc reduces reaction activity by modifying a reactive point of the ruthenium catalyst.

Examples of methods for removing the zinc responsible for reduction in catalytic activity include methods of suppressing reduction in activity by dissolving the salted-out zinc by the addition of sulfuric acid to the aqueous zinc sulfate solution. The regeneration treatment of the catalyst can also reduce the level of zinc adsorbed to ruthenium surface and produces an effect of suppressing reduction in catalytic activity.

On the other hand, the addition of sulfuric acid to the aqueous zinc sulfate solution or the regeneration treatment of the catalyst also acts to reduce the level of zinc in the catalyst effective for enhancing selectivity.

From the viewpoint mentioned above, in the first and second embodiments, it is preferred to adjust the concentration of zinc in solid matter of the ruthenium catalyst to the range described above.

If the concentration of such zinc is decreased to below 0.5% by mass, selectivity of cycloolefin is reduced. If the zinc concentration exceeds 3.5% by mass, activity is reduced and selectivity of cycloolefin tends to be reduced by influence such as influence to inhibit reaction due to too high a zinc concentration.

Specifically, in performing the addition of sulfuric acid or the regeneration treatment of the catalyst at a reaction site where nitrogen-containing components, dimethylacetamide, and dimethylamine come into the aqueous zinc sulfate solution, it is preferred to properly control the concentration of zinc contained in the ruthenium catalyst, from the viewpoint of stably keeping the activity of the catalyst and selectivity of cycloolefin.

Examples

Hereinafter, the present embodiment will be described in more detail with reference to specific Examples and Comparative Examples. However, the present invention is not limited by Examples and Comparative Examples given below by any means.

Methods for evaluating various physical properties are as described below.

[First Example]

(Nitrogen concentration in aqueous zinc sulfate solution, nitrogen concentration in monocyclic aromatic hydrocarbon, and ratio of nitrogen mass to zirconia-containing ruthenium catalyst mass)

A nitrogen concentration in an aqueous zinc sulfate solution and a monocyclic aromatic hydrocarbon was measured by a chemical luminescence method using a TS-2100V total nitrogen automatic analysis apparatus manufactured by Nittoseiko Analytech Co., Ltd. (former: Mitsubishi Chemical Analytech Co., Ltd.).

A ratio of a nitrogen mass to a zirconia-containing ruthenium catalyst mass was determined as a ratio of a total nitrogen mass concentration to a mass concentration of a total solid catalyst in the aqueous zinc sulfate solution (total nitrogen concentration in the aqueous zinc sulfate solution / mass concentration of a total solid catalyst in the aqueous zinc sulfate solution). Specifically, the reaction solution, the aqueous zinc sulfate solution, and the zirconia-containing ruthenium catalyst were extracted in a completely mixed state from the reaction system and left standing for separation. The total nitrogen concentration in the aqueous zinc sulfate solution was measured using the analysis apparatus described above. The mass concentration of the zirconia-containing ruthenium catalyst was determined as a solid concentration in the aqueous zinc sulfate solution by filtration and drying treatment, and the ratio of a nitrogen mass to a mass of the zirconia-containing ruthenium catalyst was thereby calculated.

(Dimethylamine concentration in aqueous zinc sulfate solution, dimethylamine concentration in monocyclic aromatic hydrocarbon, and ratio of dimethylamine mass to zirconia-containing ruthenium catalyst mass)

A dimethylamine concentration in the aqueous zinc sulfate solution was measured using an IC-2010 ion chromatograph apparatus from Tosoh Techno-System, Inc.

A dimethylamine concentration in the monocyclic aromatic hydrocarbon was measured using a GC-2014 gas chromatograph equipped with an FID detector, manufactured by Shimadzu Corp.

A ratio of a dimethylamine mass to a zirconia-containing ruthenium catalyst mass was calculated from the measured concentration of dimethylamine in the aqueous zinc sulfate solution extracted from the reaction system, and the solid concentration of the zirconia-containing ruthenium catalyst, in the same manner as the method for calculating the nitrogen concentration per catalyst.

(Zinc concentration in zirconia-containing ruthenium catalyst)

A zinc concentration in the zirconia-containing ruthenium catalyst was measured using a ZSX Primus II fluorescent analysis apparatus manufactured by Rigaku Holdings Corp.

In analyzing a small amount of a sample, the zirconia-containing ruthenium catalyst was dissolved in hydrochloric acid for toxic metal measurement, and a concentration of zinc dissolved in the solution was measured using an SPS3520UV-DD ICP apparatus manufactured by Hitachi High-Tech Corp.

(Average crystallite diameter of ruthenium)

An average crystallite diameter of ruthenium was determined according to the Scherrer equation from the extent of a diffraction peak at a diffraction angle $(2\theta)$ of 44° of the ruthenium metal obtained using an XRD-6100 X-ray diffraction apparatus manufactured by Shimadzu Corp.

(Conversion rate of benzene and selection rate of cyclohexene)

A 1 L high-pressure autoclave reactor was charged with the catalyst and hydrogen. While a temperature and a hydrogen pressure were set, a predetermined amount of benzene was added thereto as a monocyclic aromatic hydrocarbon at once. Reaction based on a batch method was evaluated at the predetermined reaction temperature and hydrogen pressure.

The reaction based on the batch method, also called batch reaction, is a method that involves charging an autoclave with an aqueous zinc sulfate solution containing a catalyst, setting a predetermined temperature, pressure, and time, then adding a starting material benzene once at the start of reaction, and recovering the whole amount after the completion of reaction.

After benzene addition, a portion of the reaction solution was extracted and analyzed using a gas chromatograph (GC-2014 manufactured by Shimadzu Corp.) equipped with an FID detector. A conversion rate of the reaction and a selection rate were collected over time according to the expressions (1) and (2) given below on the basis of a concentration analysis value from the experiment, and a selection rate at a conversion rate of 50% was further determined by analysis data plot.

Meanwhile, a 4 L continuous stirred tank reactor (CSTR) was charged with the catalyst, and benzene and hydrogen were continuously added thereto. Reaction was evaluated by a continuous flow method of examining a reaction process. The reaction based on the continuous flow method is a method that involves continuously adding a starting material benzene to a reactor and continuously recovering a reacted product from the reactor. This reaction can be continuously performed under constant conditions over a long period. A benzene conversion rate is adjusted by controlling a temperature, a pressure, and an amount of the catalyst as well as a benzene flow rate. In this respect, a selection rate of cyclohexene can be determined.

In the present Example, the benzene conversion rate was set to approximately 50%. As in the batch method, the reaction solution was analyzed using a gas chromatograph (GC-2014 manufactured by Shimadzu Corp.) equipped with an FID detector. The benzene conversion rate and the selection rate of cyclohexene were determined according to the expressions (1) and (2) given below on the basis of a concentration analysis value from the experiment.

Although the reaction performance of the continuous stirred tank reactor is theoretically lower than the selection rate of the reaction determined for the batch reactor, industrial production is performed in this continuous stirred tank reaction format because of its high production efficiency. Examples of the batch evaluation and Examples of the continuous flow method were summarized in Tables 1 and 2, respectively, because evaluation values differ therebetween.

$$\text{Conversion rate of benzene (\%)} = \frac{\text{The number of moles of benzene consumed through reaction}}{\text{The number of moles of benzene supplied to reaction}} \times 100 \qquad (1)$$

$$\text{Selection rate of cyclohexene (\%)} = \frac{\text{The number of moles of cyclohexene produced through reaction}}{\text{The number of moles of cyclohexane produced through reaction} + \text{The number of moles of cyclohexane produced through reaction}} \times 100 \qquad (2)$$

[Example 1-1]

(Preparation of catalyst)

20 g of ruthenium chloride hydrate (containing 40% by mass of ruthenium) and 8 g of zinc chloride were added with stirring to 500 mL of distilled water warmed to 80°C, and dissolved therein. 100 mL of a 5 N aqueous sodium hydroxide solution was added thereto, and stirring was further continued at 80°C for 2 hours to obtain a black precipitated solid. The solution containing the solid was cooled and filtered, and the recovered solid was added into 500 mL of a 1 N aqueous sodium hydroxide solution, followed by three repetitions of alkali washing and filtration operation. Then, water washing and filtration were further repeated five times using 500 mL of distilled water. Subsequently, the solid was added to a measuring cylinder containing an aqueous zinc sulfate solution provided in advance, and adjusted to a zinc sulfate concentration of 0.62 mol/L, and 500 mL of the resulting solution was added to a 1 L autoclave. Further, 45 g of a zirconia powder was added thereto. The 1 L autoclave was set and subjected to increase in temperature and pressure with stirring. Reduction treatment for 5 hours was carried out with 5 MPa of hydrogen having a temperature of 150°C.

Then, the 1 L autoclave was cooled, and the solution containing a ruthenium catalyst containing reduced zirconia was recovered into a beaker, which was then left standing, followed by operation of removing a supernatant by decantation. Further, distilled water was added thereto, and the beaker was stirred and left standing, followed by five repetitions of operation of exchanging a supernatant by decantation to collect 52.8 g (based on a dry mass) of a wet ruthenium catalyst containing zirconia.

The average crystallite diameter of ruthenium in the zirconia-containing ruthenium catalyst was 4.6 nm.

(Partial hydrogenation of benzene)

A 1 L autoclave was charged with 280 mL of a solution that contained 12 g (based on a dry mass) of the zirconia-containing ruthenium catalyst and had a dimethylamine concentration adjusted to 45 mg/L and a zinc sulfate concentration adjusted to 0.62 mol/L.

The autoclave was purged with hydrogen with stirring and heated to 145°C. Then, hydrogen was further injected thereto, and the reaction pretreatment of a catalyst slurry was performed while the total pressure was kept at 5 MPa for 22 hours.

Subsequently, the pressure of the autoclave was temporarily lowered to 3 MPa. Then, 140 mL of benzene was injected thereto together with hydrogen and reacted with high-speed stirring at a total pressure of 5 MPa.

A conversion rate of benzene and a selection rate of cyclohexene were determined from results of extracting the reaction solution over time and compositionally analyzing the liquid phase by gas chromatography.

After reaction, the reaction product and the catalyst slurry were recovered from the autoclave, and a nitrogen concentration in the aqueous zinc sulfate solution, a ratio of a nitrogen mass to a mass of the added zirconia-containing ruthenium catalyst, and a concentration of zinc contained in the zirconia-containing ruthenium catalyst were measured.

Table 1 below shows each analysis value and the selection rate of cyclohexene at a benzene conversion rate of 50%.

[Example 1-2]

A 1 L autoclave was charged with 280 mL of a solution that contained 12 g (based on a dry mass) of the zirconia-containing ruthenium catalyst prepared in Example 1-1 and 1.15 g of 40% by mass of dimethylamine and had a zinc sulfate concentration adjusted to 0.62 mol/L.

The autoclave was purged with hydrogen with stirring and heated to 145°C. Then, hydrogen was further injected thereto, and the reaction pretreatment of a catalyst slurry was performed while the total pressure was kept at 5 MPa for 22 hours.

Subsequently, the autoclave was cooled, depressurized, and opened, and 0.52 g of 96% by mass of sulfuric acid was added thereto. Hydrogen was injected thereto again, and the autoclave was stirred at 145°C at a total pressure of 5 MPa for 1 hour. Then, the pressure of the autoclave was temporarily lowered to 3 MPa. Then, 140 mL of benzene was injected thereto together with hydrogen and reacted with high-speed stirring at a total pressure of 5 MPa.

A conversion rate of benzene and a selection rate of cyclohexene were determined from results of extracting the reaction solution over time and compositionally analyzing the liquid phase by gas chromatography.

After reaction, the reaction product and the catalyst slurry were recovered from the autoclave, and a nitrogen concentration in the aqueous zinc sulfate solution, a ratio of a nitrogen mass to a mass of the added catalyst, and a concentration of zinc contained in the zirconia-containing ruthenium catalyst were measured.

Table 1 below shows each analysis value and the selection rate of cyclohexene at a benzene conversion rate of 50%.

[Example 1-3]

A 1 L autoclave was charged with 280 mL of a solution that contained 12 g (based on a dry mass) of the zirconia-containing ruthenium catalyst prepared in Example 1-1 and 3.03 g of 40% by mass of dimethylamine and had a zinc sulfate concentration adjusted to 0.62 mol/L.

The autoclave was purged with hydrogen with stirring and heated to 145°C. Then, hydrogen was further injected thereto, and the reaction pretreatment of a catalyst slurry was performed while the total pressure was kept at 5 MPa for 22 hours.

Subsequently, the autoclave was cooled, depressurized, and opened, and 1.5 g of 96% by mass of sulfuric acid was added thereto. Hydrogen was injected thereto again, and the autoclave was stirred at 145°C at a total pressure of 5 MPa for 1 hour. Then, the pressure of the autoclave was temporarily lowered to 3 MPa. Then, 140 mL of benzene was injected thereto together with hydrogen and reacted with high-speed stirring at a total pressure of 5 MPa.

A conversion rate of benzene and a selection rate of cyclohexene were determined from results of extracting the reaction solution over time and compositionally analyzing the liquid phase by gas chromatography.

After reaction, the reaction product and the catalyst slurry were recovered from the autoclave, and a nitrogen concentration in the aqueous zinc sulfate solution, a ratio of a nitrogen mass to a mass of the added catalyst, and a concentration of zinc contained in the zirconia-containing ruthenium catalyst were measured.

Table 1 below shows each analysis value and the selection rate of cyclohexene at a benzene conversion rate of 50%.

[Example 1-4]

A 1 L autoclave was charged with 280 mL of a solution that contained 12 g (based on a dry mass) of the zirconia-containing ruthenium catalyst prepared in Example 1-1 and 6.08 g of 40% by mass of dimethylamine and had a zinc sulfate concentration adjusted to 0.62 mol/L.

The autoclave was purged with hydrogen with stirring and heated to 145°C. Then, hydrogen was further injected thereto, and the reaction pretreatment of a catalyst slurry was performed while the total pressure was kept at 5 MPa for 22 hours.

Subsequently, the autoclave was cooled, depressurized, and opened, and 2.81 g of 96% by mass of sulfuric acid was added thereto. Hydrogen was injected thereto again, and the autoclave was stirred at 145°C at a total pressure of 5 MPa for 1 hour. Then, the pressure of the autoclave was temporarily lowered to 3 MPa. Then, 140 mL of benzene was injected thereto together with hydrogen and reacted with high-speed stirring at a total pressure of 5 MPa.

A conversion rate of benzene and a selection rate of cyclohexene were determined from results of extracting the reaction solution over time and compositionally analyzing the liquid phase by gas chromatography.

After reaction, the reaction product and the catalyst slurry were recovered from the autoclave, and a nitrogen concentration in the aqueous zinc sulfate solution, a ratio of a nitrogen mass to a mass of the added catalyst, and a concentration of zinc contained in the zirconia-containing ruthenium catalyst were measured.

Table 1 below shows each analysis value and the selection rate of cyclohexene at a benzene conversion rate of 50%.

[Comparative Example 1-1]

53.1 g (based on a dry mass) of the zirconia-containing ruthenium catalyst prepared in the same manner as in Example 1-1 was recovered.

A 1 L autoclave was charged with 280 mL of a solution that contained 12 g of the zirconia-containing ruthenium catalyst and 19.56 g of 40% by mass of dimethylamine and had a zinc sulfate concentration adjusted to 0.62 mol/L.

The autoclave was purged with hydrogen with stirring and heated to 145°C. Then, hydrogen was further injected thereto, and the reaction pretreatment of a catalyst slurry was performed while the total pressure was kept at 5 MPa for 22 hours.

Subsequently, the autoclave was cooled, depressurized, and opened, and 8.73 g of 96% by mass of sulfuric acid was added thereto. Hydrogen was injected thereto again, and the autoclave was stirred at 145°C at a total pressure of 5 MPa for 1 hour. Then, the pressure of the autoclave was temporarily lowered to 3 MPa. Then, 140 mL of benzene was injected thereto together with hydrogen and reacted with high-speed stirring at a total pressure of 5 MPa.

A conversion rate of benzene and a selection rate of cyclohexene were determined from results of extracting the reaction solution over time and compositionally analyzing the liquid phase by gas chromatography.

After reaction, the reaction product and the catalyst slurry were recovered from the autoclave, and a nitrogen concentration in the aqueous zinc sulfate solution, a ratio of a nitrogen mass to a mass of the added catalyst, and a concentration of zinc contained in the zirconia-containing ruthenium catalyst were measured.

Table 1 below shows each analysis value and the selection rate of cyclohexene at a benzene conversion rate of 50%.

[Comparative Example 1-2]

The zirconia-containing ruthenium catalyst and the reaction solution reacted in Example 1-4 were recovered from the autoclave. An aromatic hydrocarbon component in the reaction solution was removed, and the catalyst slurry was further treated by nitrogen blow to the extent that the odor of aromatic hydrocarbon became imperceptible.

240 mL of the catalyst slurry was collected in a completely mixed state, and 10.67 g of 40% by mass of dimethylamine was added thereto. A 1 L autoclave was charged with the mixture.

The autoclave was purged with hydrogen with stirring and heated to 145°C. Then, hydrogen was further injected thereto, and the reaction pretreatment of a catalyst slurry was performed while the total pressure was kept at 5 MPa for 22 hours.

Subsequently, the autoclave was cooled, depressurized, and opened, and 4.71 g of 96% by mass of sulfuric acid was added thereto. Hydrogen was injected thereto again, and the autoclave was stirred at 145°C at a total pressure of 5 MPa for 1 hour. Then, the pressure of the autoclave was temporarily lowered to 3 MPa. Then, 120 mL of benzene was injected thereto together with hydrogen and reacted with high-speed stirring at a total pressure of 5 MPa.

A conversion rate of benzene and a selection rate of cyclohexene were determined from results of extracting the reaction solution over time and compositionally analyzing the liquid phase by gas chromatography.

After reaction, the reaction product and the catalyst slurry were recovered from the autoclave, and a nitrogen concentration in the aqueous zinc sulfate solution, a ratio of a nitrogen mass to a mass of the catalyst in the reactor, and a concentration of zinc contained in the zirconia-containing ruthenium catalyst were measured.

Table 1 below shows each analysis value and the selection rate of cyclohexene at a benzene conversion rate of 50%.

[Comparative Example 1-3]

The zirconia-containing ruthenium catalyst and the reaction solution reacted in Comparative Example 1-2 were recovered from the autoclave. An aromatic hydrocarbon component in the reaction solution was removed, and the catalyst slurry was further treated by nitrogen blow to the extent that the odor of aromatic hydrocarbon became imperceptible.

220 mL of the catalyst slurry was collected in a completely mixed state, and 0.061 g of 96% by mass of sulfuric acid was added thereto. A 1 L autoclave was charged with the mixture.

Hydrogen was injected thereto again, and the autoclave was stirred at 145°C at a total pressure of 5 MPa for 1 hour. Then, the pressure of the autoclave was temporarily lowered to 3 MPa. Then, 110 mL of benzene was injected thereto together with hydrogen and reacted with high-speed stirring at a total pressure of 5 MPa.

A conversion rate of benzene and a selection rate of cyclohexene were determined from results of extracting the reaction solution over time and compositionally analyzing the liquid phase by gas chromatography.

After reaction, the reaction product and the catalyst slurry were recovered from the autoclave, and a nitrogen concentration in the aqueous zinc sulfate solution, a ratio of a nitrogen mass to a mass of the catalyst in the reactor, and a concentration of zinc contained in the zirconia-containing ruthenium catalyst were measured.

Table 1 below shows each analysis value and the selection rate of cyclohexene at a benzene conversion rate of 50%.

[Example 1-5]

The zirconia-containing ruthenium catalyst and the reaction solution reacted in Comparative Example 1-1 were recovered from the autoclave. An aromatic hydrocarbon component in the reaction solution was removed, and the catalyst slurry was further treated by nitrogen blow to the extent that the odor of aromatic hydrocarbon became imperceptible.

240 mL of the catalyst slurry was collected in a completely mixed state and left standing to precipitate catalyst solid matter. Then, 140 mL of a supernatant was removed, and the residue was diluted to 280 mL by fresh addition of a 0.62 mol/L aqueous zinc sulfate solution.

Subsequently, the catalyst slurry was stirred for 5 minutes and left standing to precipitate catalyst solid matter. Then, 180 mL of a supernatant was removed, and the residue was diluted to 280 mL by fresh addition of a 0.62 mol/L aqueous zinc sulfate solution. This operation was repeated twice.

A 1 L autoclave was charged with 280 mL of the treated catalyst slurry, purged with hydrogen with stirring, and heated to 145°C. Then, hydrogen was further injected thereto, and the pretreatment of a catalyst was performed while the total pressure was kept at 5 MPa for 22 hours.

Subsequently, the pressure of the autoclave was temporarily lowered to 3 MPa. Then, 140 mL of benzene was injected thereto together with hydrogen and reacted with high-speed stirring at a total pressure of 5 MPa.

A conversion rate of benzene and a selection rate of cyclohexene were determined from results of extracting the reaction solution over time and compositionally analyzing the liquid phase by gas chromatography.

After reaction, the reaction product and the catalyst slurry were recovered from the autoclave, and a nitrogen concentration in the aqueous zinc sulfate solution, a ratio of a nitrogen mass to a mass of the catalyst in the reactor, and a concentration of zinc contained in the zirconia-containing ruthenium catalyst were measured.

Table 1 below shows each analysis value and the selection rate of cyclohexene at a benzene conversion rate of 50%.

[Table 1]

| | | Total nitrogen concentration | | Dimethylamine (DMA) concentration | | Cyclohexene selection rate (%) | Zn concentration in catalyst (% by mass) |
|---|---|---|---|---|---|---|---|
| | | Concentration in aqueous zinc sulfate solution (mg/L) | Ratio of nitrogen mass to catalyst mass (times) | Concentration in aqueous zinc sulfate solution (mg/L) | Ratio of dimethylamine mass to catalyst mass (times) | | |
| Example 1-1 | DMA was added to catalyst slurry before reaction | 14 | $3.3 \times 10^{-4}$ | 45 | $1.1 \times 10^{-3}$ | 84.8 | 1.60 |
| Example 1-2 | DMA was added to catalyst slurry before reaction | 502 | $1.2 \times 10^{-2}$ | 1649 | $3.8 \times 10^{-2}$ | 84.5 | 1.50 |
| Example 1-3 | DMA was added to catalyst slurry before reaction | 1303 | $3.0 \times 10^{-2}$ | 4325 | 0.10 | 84.5 | 0.69 |
| Example 1-4 | DMA was added to catalyst slurry before reaction | 2657 | $6.2 \times 10^{-2}$ | 8685 | 0.20 | 83.7 | 0.73 |
| Comparative Example 1-1 | DMA was added to catalyst slurry before reaction | 8504 | 0.20 | 27938 | 0.65 | 78.3 | 1.05 |
| Comparative Example 1-2 | Oil matter in reaction solution of Example 1-4 was removed, and additional DMA was added | 7796 | 0.19 | 25560 | 0.63 | 78.0 | 0.86 |
| Comparative Example 1-3 | Sulfuric acid was added to catalyst slurry of Comparative Example 1-2 | 7768 | 0.24 | 25468 | 0.79 | 76.1 | 0.42 |
| Example 1-5 | Oil matter in reaction solution of Comparative Example 1-1 was removed, and a portion of aqueous zinc sulfate solution was replaced | 387 | $1.1 \times 10^{-2}$ | 1269 | $3.5 \times 10^{-2}$ | 84.6 | 1.01 |

[Example 1-6]

A continuous stirred tank reactor was charged with 1200 mL of a solution that contained 50 g (based on a dry mass) of the zirconia-containing ruthenium catalyst prepared in the same manner as in Example 1-1 and had a zinc sulfate concentration of 0.62 mol/L.

This continuous stirred tank reactor has an oil-water separation tank as an attached tank and is provided with Teflon(R) coating on the inner surface.

Subsequently, benzene containing 0.1 mg/L dimethylamine was supplied at 1.2 L/hr to the continuous stirred tank reactor under hydrogen pressure of 5 MPa at 145°C to continuously perform the partial hydrogenation reaction of benzene.

In this operation, water was supplied such that a water phase containing the zirconia-containing ruthenium catalyst in the reaction system always had constant composition. A reaction product composed of benzene, cyclohexene, and cyclohexane was continuously recovered from the oil-water separation tank.

The volume ratio between the water phase and an oil phase in the reactor was kept constant at water phase:oil phase = 2:1.

A conversion rate of benzene and a selection rate of cyclohexene were determined from results of collecting a reaction solution coming out of the oil-water separation tank after 22 hours from the start of reaction and compositionally analyzing the liquid phase by gas chromatography.

A catalyst slurry was collected following the sampling of the reaction solution from a catalyst sampling line established in the tank reactor, and a nitrogen concentration in the aqueous zinc sulfate solution, a ratio of a nitrogen mass to a mass of the catalyst in the reactor, and a concentration of zinc contained in the zirconia-containing ruthenium catalyst were measured.

Table 2 below shows each analysis value, the conversion rate of benzene, and the selection rate of cyclohexene.

[Example 1-7]

The reaction of Example 1-6 was continued. A conversion rate of benzene and a selection rate of cyclohexene were determined from results of collecting a reaction solution coming out of the oil-water separation tank after 116 hours from the start of reaction and compositionally analyzing the liquid phase by gas chromatography. As a result, the reaction performance was a benzene conversion rate of 45.7% and a cyclohexene selection rate of 78.8%.

The benzene conversion rate was decreased based on the reaction performance obtained at a reaction time of 22 hours. Therefore, the supply rate of benzene was lowered with a benzene conversion rate of 50% as a guideline, and a stability time was secured for 2 hours. Then, the reaction solution was collected again, and reaction performance was determined.

A catalyst slurry was collected, and a nitrogen concentration in the aqueous zinc sulfate solution, a ratio of a nitrogen mass to a mass of the catalyst in the reactor, and a concentration of zinc contained in the zirconia-containing ruthenium catalyst were measured.

Table 2 below shows each analysis value, the conversion rate of benzene, and the selection rate of cyclohexene.

[Example 1-8]

A continuous stirred tank reactor was charged with 1200 mL of a solution that contained 50 g (based on a dry mass) of the zirconia-containing ruthenium catalyst prepared in the same manner as in Example 1-1 and had a zinc sulfate concentration adjusted to 0.62 mol/L.

Subsequently, benzene containing 10 mg/L dimethylamine was supplied at 1.2 L/hr to the continuous stirred tank reactor under hydrogen pressure of 5 MPa at 145°C to continuously perform the partial hydrogenation reaction of benzene.

After 20 hours from the start of reaction, 0.3 g of 96% by mass of sulfuric acid was added to the reactor through a supply line of replenished water in the reaction system. A conversion rate of benzene and a selection rate of cyclohexene were determined from results of collecting a reaction solution coming out of the oil-water separation tank after 22 hours from the start of reaction and compositionally analyzing the liquid phase by gas chromatography.

A catalyst slurry was collected following the sampling of the reaction solution from a catalyst sampling line established in the continuous stirred tank reactor, and a nitrogen concentration in the aqueous zinc sulfate solution, a ratio of a nitrogen mass to a mass of the catalyst in the reactor, and a concentration of zinc contained in the zirconia-containing ruthenium catalyst were measured.

Table 2 below shows each analysis value, the conversion rate of benzene, and the selection rate of cyclohexene.

[Example 1-9]

The reaction of Example 1-8 was continued. After 24 hours from the start of reaction, 600 mL of a catalyst slurry containing the reaction solution was extracted from the continuous stirred tank reactor, while the continuous stirred tank reactor was charged with 400 mL of the same catalyst slurry containing zinc sulfate as that initially added in Example 1-8.

An aromatic hydrocarbon component was removed from the extracted catalyst slurry containing the reaction solution, and the catalyst slurry was further treated by nitrogen blow to the extent that the odor of aromatic hydrocarbon became imperceptible.

Subsequently, nitrogen containing 7% by volume of oxygen was contacted therewith at 70°C for 1 hour. Then, the reactor was purged with nitrogen and further purged with hydrogen.

Then, the reactor was heated to 140°C and stirred for 4 hours while the internal pressure of the system was kept at 0.5 MPa in a hydrogen atmosphere. Operation of restoring catalytic activity was performed using the whole amount of the catalyst slurry.

In this way, operation of extracting 400 mL of the catalyst slurry from the reactor as mentioned above, restoring catalytic activity, and bringing the slurry thus treated back to the reactor was continued at a frequency of once per day while the reaction was continued.

The catalyst slurry treated by the activity restoration operation was brought back to the reactor after being supplemented with 0.41 g of 96% by mass of sulfuric acid.

A conversion rate of benzene and a selection rate of cyclohexene were determined from results of collecting a reaction solution coming out of the oil-water separation tank after 118 hours from the start of reaction and compositionally analyzing the liquid phase by gas chromatography.

A catalyst slurry was collected following the sampling of the reaction solution from a catalyst sampling line established in the continuous stirred tank reactor, and a nitrogen concentration in the aqueous zinc sulfate solution, a ratio of a nitrogen mass to a mass of the catalyst in the reactor, and a concentration of zinc contained in the zirconia-containing ruthenium catalyst were measured.

Table 2 below shows each analysis value, the conversion rate of benzene, and the selection rate of cyclohexene.

[Example 1-10]

A continuous stirred tank reactor was charged with 1200 mL of a solution that contained 50 g (based on a dry mass) of the zirconia-containing ruthenium catalyst prepared in the same manner as in Example 1-1 and had a zinc sulfate concentration adjusted to 0.62 mol/L.

Subsequently, benzene containing 50 mg/L dimethylamine was supplied at 1.2 L/hr to the continuous stirred tank reactor under hydrogen pressure of 5 MPa at 145°C to continuously perform the partial hydrogenation reaction of benzene.

After 20 hours from the start of reaction, 1.5 g of 96% by mass of sulfuric acid was added to the reactor through a supply line of replenished water in the reaction system. A conversion rate of benzene and a selection rate of cyclohexene were determined from results of collecting a reaction solution coming out of the oil-water separation tank after 22 hours from the start of reaction and compositionally analyzing the liquid phase by gas chromatography.

A catalyst slurry was collected following the sampling of the reaction solution from a catalyst sampling line established in the continuous stirred tank reactor, and a nitrogen concentration in the aqueous zinc sulfate solution, a ratio of a nitrogen mass to a mass of the catalyst in the reactor, and a concentration of zinc contained in the zirconia-containing ruthenium catalyst were measured.

Table 2 below shows each analysis value, the conversion rate of benzene, and the selection rate of cyclohexene.

[Example 1-11]

The reaction of Example 1-10 was continued. After 24 hours from the start of reaction, 600 mL of a catalyst slurry containing the reaction solution was extracted from the continuous stirred tank reactor, while the continuous stirred tank reactor was charged with 400 mL of the same catalyst slurry containing zinc sulfate as that initially added in Example 1-10.

An aromatic hydrocarbon component was removed from the extracted catalyst slurry containing the reaction solution, and the catalyst slurry was further treated by nitrogen blow to the extent that the odor of aromatic hydrocarbon became imperceptible.

Subsequently, nitrogen containing 7% by volume of oxygen was contacted therewith at 70°C for 1 hour. Then, the reactor was purged with nitrogen and further purged with hydrogen.

Then, the reactor was heated to 140°C and stirred for 4 hours while the internal pressure of the system was kept at 0.5 MPa in a hydrogen atmosphere. Operation of restoring catalytic activity was performed using the whole amount of the catalyst slurry.

In this way, operation of extracting 400 mL of the catalyst slurry from the reactor as mentioned above, restoring catalytic activity, and bringing the slurry thus treated back to the reactor was continued at a frequency of once per day while the reaction was continued.

The catalyst slurry treated by the activity restoration operation was brought back to the reactor after being supplemented with 2.1 g of 96% by mass of sulfuric acid.

A conversion rate of benzene and a selection rate of cyclohexene were determined from results of collecting a reaction solution coming out of the oil-water separation tank after 118 hours from the start of reaction and compositionally analyzing the liquid phase by gas chromatography.

A catalyst slurry was collected following the sampling of the reaction solution from a catalyst sampling line established in the continuous stirred tank reactor, and a nitrogen concentration in the aqueous zinc sulfate solution, a ratio of a nitrogen mass to a mass of the catalyst in the reactor, and a concentration of zinc contained in the zirconia-containing ruthenium catalyst were measured.

Table 2 below shows each analysis value, the conversion rate of benzene, and the selection rate of cyclohexene.

[Example 1-12]

A continuous stirred tank reactor was charged with 1200 mL of a solution that contained 50 g (based on a dry mass) of the zirconia-containing ruthenium catalyst prepared in the same manner as in Example 1-1 and had a zinc sulfate concentration adjusted to 0.62 mol/L.

Subsequently, benzene containing 100 mg/L dimethylamine was supplied at 1.2 L/hr to the continuous stirred tank reactor under hydrogen pressure of 5 MPa at 145°C to continuously perform the partial hydrogenation reaction of benzene.

After 20 hours from the start of reaction, 3 g of 96% by mass of sulfuric acid was added to the reactor through a supply line of replenished water in the reaction system. A conversion rate of benzene and a selection rate of cyclohexene were determined from results of collecting a reaction solution coming out of the oil-water separation tank after 22 hours from the start of reaction and compositionally analyzing the liquid phase by gas chromatography.

A catalyst slurry was collected following the sampling of the reaction solution from a catalyst sampling line established in the continuous stirred tank reactor, and a nitrogen concentration in the aqueous zinc sulfate solution, a ratio of a nitrogen mass to a mass of the catalyst in the reactor, and a concentration of zinc contained in the zirconia-containing ruthenium catalyst were measured.

Table 2 below shows each analysis value, the conversion rate of benzene, and the selection rate of cyclohexene.

[Example 1-13]

The reaction of Example 1-12 was continued. After 24 hours from the start of reaction, 600 mL of a catalyst slurry containing the reaction solution was extracted from the continuous stirred tank reactor, while the continuous stirred tank reactor was charged with 400 mL of the same catalyst slurry containing zinc sulfate as that initially added in Example 1-10.

An aromatic hydrocarbon component was removed from the extracted catalyst slurry containing the reaction solution, and the catalyst slurry was further treated by nitrogen blow to the extent that the odor of aromatic hydrocarbon became imperceptible.

Subsequently, nitrogen containing 7% by volume of oxygen was contacted therewith at 50°C for 1 hour. Then, the reactor was purged with nitrogen and further purged with hydrogen.

Then, the reactor was heated to 140°C and stirred for 4 hours while the internal pressure of the system was kept at 0.5 MPa in a hydrogen atmosphere. Operation of restoring catalytic activity was performed using the whole amount of the catalyst slurry.

In this way, operation of extracting 400 mL of the catalyst slurry from the reactor as mentioned above, restoring catalytic activity, and bringing the slurry thus treated back to the reactor was continued at a frequency of once per day while the reaction was continued.

The catalyst slurry treated by the activity restoration operation was brought back to the reactor after being supplemented with 4 g of 96% by mass of sulfuric acid.

A conversion rate of benzene and a selection rate of cyclohexene were determined from results of collecting a reaction solution coming out of the oil-water separation tank after 118 hours from the start of reaction and compositionally analyzing the liquid phase by gas chromatography.

A catalyst slurry was collected following the sampling of the reaction solution from a catalyst sampling line established in the continuous stirred tank reactor, and a nitrogen concentration in the aqueous zinc sulfate solution, a ratio of a nitrogen mass to a mass of the catalyst in the reactor, and a concentration of zinc contained in the zirconia-containing ruthenium catalyst were measured.

Table 2 below shows each analysis value, the conversion rate of benzene, and the selection rate of cyclohexene.

[Comparative Example 1-4]

The reaction of Example 1-13 was continued. Operation of extracting 400 mL of a catalyst slurry containing the reaction solution, restoring catalytic activity, and bringing 400 mL of the slurry thus treated back to the reactor was continued at a frequency of once per day.

The catalyst slurry treated by the activity restoration operation was brought back to the reactor after being supplemented with 3.26 g of 96% by mass of sulfuric acid.

A conversion rate of benzene and a selection rate of cyclohexene were determined from results of collecting a reaction solution coming out of the oil-water separation tank after 310 hours from the start of reaction and compositionally analyzing the liquid phase by gas chromatography.

A catalyst slurry was collected following the sampling of the reaction solution from a catalyst sampling line established in the continuous stirred tank reactor, and a nitrogen concentration in the aqueous zinc sulfate solution, a ratio of a nitrogen mass to a mass of the catalyst in the reactor, and a concentration of zinc contained in the zirconia-containing ruthenium catalyst were measured.

Table 2 below shows each analysis value, the conversion rate of benzene, and the selection rate of cyclohexene.

[Comparative Example 1-5]

A continuous stirred tank reactor was charged with 1200 mL of a solution that contained 50 g (based on a dry mass) of the zirconia-containing ruthenium catalyst prepared in the same manner as in Example 1-1 and had a zinc sulfate concentration adjusted to 0.62 mol/L.

Subsequently, benzene containing 200 mg/L dimethylamine was supplied at 1.2 L/hr to the continuous stirred tank reactor under hydrogen pressure of 5 MPa at 145°C to continuously perform the partial hydrogenation reaction of benzene.

After 24 hours from the start of reaction, 600 mL of a catalyst slurry containing the reaction solution was extracted from the continuous stirred tank reactor, while the continuous stirred tank reactor was charged with 400 mL of the same catalyst slurry containing zinc sulfate as that initially added in Example 1-1.

An aromatic hydrocarbon component was removed from the extracted catalyst slurry containing the reaction solution, and the catalyst slurry was further treated by nitrogen blow to the extent that the odor of aromatic hydrocarbon became imperceptible.

Subsequently, nitrogen containing 7% by volume of oxygen was contacted therewith at 50°C for 1 hour. Then, the reactor was purged with nitrogen and further purged with hydrogen.

Then, the reactor was heated to 140°C and stirred for 4 hours while the internal pressure of the system was kept at 0.5 MPa in a hydrogen atmosphere. Operation of restoring catalytic activity was performed using the whole amount of the catalyst slurry.

In this way, operation of extracting 400 mL of the catalyst slurry from the reactor as mentioned above, restoring catalytic activity, and bringing the slurry thus treated back to the reactor was continued at a frequency of once per day while the reaction was continued.

The catalyst slurry treated by the activity restoration operation was brought back to the reactor after being supplemented with 8.1 g of 96% by mass of sulfuric acid. This operation was performed a total of three times.

A conversion rate of benzene and a selection rate of cyclohexene were determined from results of collecting a reaction solution coming out of the oil-water separation tank after 118 hours from the start of reaction and compositionally analyzing the liquid phase by gas chromatography.

A catalyst slurry was collected following the sampling of the reaction solution from a catalyst sampling line established in the continuous stirred tank reactor, and a nitrogen concentration in the aqueous zinc sulfate solution, a ratio of a nitrogen mass to a mass of the catalyst in the reactor, and a concentration of zinc contained in the zirconia-containing ruthenium catalyst were measured.

Table 2 below shows each analysis value, the conversion rate of benzene, and the selection rate of cyclohexene.

[Example 1-14]

The reaction of Comparative Example 1-5 was continued and shifted to benzene containing 0.1 mg/L dimethylamine since 120 hours from the start of reaction. Further, operation of extracting 400 mL of a catalyst slurry containing the reaction solution, restoring catalytic activity, and bringing 400 mL of the slurry thus treated back to the reactor was continued at a frequency of once per day.

The catalyst slurry brought back to the reactor since 120 hours from the start of reaction was a solution extracted from the reactor, which was used after being treated until a nitrogen concentration was no longer detected. Specifically, the catalyst slurry was treated to the extent that the odor of an aromatic hydrocarbon component became imperceptible, followed by treatment with nitrogen containing oxygen, in the same manner as in Comparative Example 1-5. Subsequently, a nitrogen concentration was decreased by repetitions of operation of removing a supernatant by decantation at room temperature and adding a freshly prepared 0.62 mol/L aqueous zinc sulfate solution. Then, 400 mL of the treated catalyst slurry was heated to 140°C, and the reactor was heated to 140°C and stirred for 4 hours while the internal pressure of the system was kept at 0.5 MPa in a hydrogen atmosphere. The catalyst slurry treated by the restoration operation was secured and used at the time of catalyst addition.

A conversion rate of benzene and a selection rate of cyclohexene were determined from results of collecting a reaction solution coming out of the oil-water separation tank after 310 hours from the start of reaction and compositionally analyzing the liquid phase by gas chromatography.

A catalyst slurry was collected following the sampling of the reaction solution from a catalyst sampling line established in the tank reactor, and a nitrogen concentration in the aqueous zinc sulfate solution, a ratio of a nitrogen mass to a mass of the catalyst in the reactor, and a concentration of zinc contained in the zirconia-containing ruthenium catalyst were measured.

Table 2 below shows each analysis value, the conversion rate of benzene, and the selection rate of cyclohexene.

[Table 2]

| | Total nitrogen concentration | | | Dimethylamine (DMA) concentration | | | Reaction time (Hr) | Benzene conversion rate (%) | Cyclohexene selection rate (%) | Zn concentration in catalyst (% by mass) |
|---|---|---|---|---|---|---|---|---|---|---|
| | Concentration in aqueous zinc sulfate solution (mg/L) | Concentration in starting material benzene (mg/L) | Ratio of nitrogen mass to catalyst mass (times) | Concentration in aqueous zinc sulfate solution (mg/L) | Concentration in starting material benzene (mg/L) | Ratio of dimethylamine mass to catalyst mass (times) | | | | |
| Example 1-6<br>DMA was added to starting material benzene | 0.64 | 0.03 | $1.5 \times 10^{-5}$ | 2.06 | 0.1 | $4.9 \times 10^{-5}$ | 22 | 51.2 | 75.3 | 1.55 |
| Example 1-7<br>Reaction time of Example 1-6 was extended | 3.4 | 0.03 | $8.2 \times 10^{-5}$ | 10.9 | 0.1 | $2.6 \times 10^{-4}$ | 118 | 50.6 | 75.8 | 1.42 |
| Example 1-8<br>DMA was added to starting material benzene | 64 | 3.0 | $1.5 \times 10^{-3}$ | 206 | 10 | $4.9 \times 10^{-3}$ | 22 | 52.5 | 74.6 | 1.37 |
| Example 1-9<br>Reaction time of Example 1-8 was extended | 270 | 3.0 | $4.9 \times 10^{-3}$ | 869 | 10 | $1.6 \times 10^{-2}$ | 118 | 52.0 | 75.3 | 1.23 |
| Example 1-10<br>DMA was added to starting material benzene | 321 | 15 | $7.7 \times 10^{-3}$ | 1033 | 50 | $2.5 \times 10^{-2}$ | 22 | 51.7 | 74.4 | 1.28 |
| Example 1-11<br>Reaction time of Example 1-10 was extended | 1350 | 15 | $2.4 \times 10^{-2}$ | 4346 | 50 | $7.8 \times 10^{-2}$ | 118 | 52.6 | 74.9 | 0.70 |
| Example 1-12<br>DMA was added to starting material benzene | 642 | 30 | $1.5 \times 10^{-2}$ | 2067 | 100 | $5.0 \times 10^{-2}$ | 22 | 52.4 | 74.1 | 1.31 |

(continued)

| | | Total nitrogen concentration | | | Dimethylamine (DMA) concentration | | | Reaction time (Hr) | Benzene conversion rate (%) | Cyclohexene selection rate (%) | Zn concentration in catalyst (% by mass) |
|---|---|---|---|---|---|---|---|---|---|---|---|
| | | Concentration in aqueous zinc sulfate solution (mg/L) | Concentration in starting material benzene (mg/L) | Ratio of nitrogen mass to catalyst mass (times) | Concentration in aqueous zinc sulfate solution (mg/L) | Concentration in starting material benzene (mg/L) | Ratio of dimethylamine mass to catalyst mass (times) | | | | |
| Example 1-13 | Reaction time of Example 1-12 was extended | 2701 | 30 | $4.9 \times 10^{-2}$ | 8696 | 100 | 0.16 | 118 | 50.8 | 73.7 | 1.10 |
| Comparative Example 1-4 | Reaction time of Example 1-13 was extended | 6904 | 30 | 0.12 | 22228 | 100 | 0.40 | 310 | 51.3 | 68.7 | 0.84 |
| Comparative Example 1-5 | DMA was added to starting material benzene | 5402 | 61 | $9.7 \times 10^{-2}$ | 17392 | 200 | 0.31 | 118 | 51.5 | 69.2 | 0.88 |
| Example 1-14 | A portion of aqueous zinc sulfate solution of Comparative Example 1-4 was replaced | 217 | 0.03 | 0.4 | 699 | 0.1 | $1.3 \times 10^{-2}$ | 310 | 50.9 | 74.2 | 0.82 |

EP 4 567 017 A1

[Second Example]

(Acetic acid concentration)

<Acetic acid concentration in aqueous zinc sulfate solution>

An acetic acid concentration in an aqueous zinc sulfate solution was measured using an IC-2010 ion chromatograph apparatus from Tosoh Techno-System, Inc.

The acetic acid concentration, in the case of 1 mg/L or lower, was determined by subtracting the amount of acetic acid that was not retained in the aqueous zinc sulfate solution by dissolution in a monocyclic aromatic hydrocarbon and a reaction solution from the amount of acetic acid added into the aqueous zinc sulfate solution.

Specifically, the acetic acid concentration was determined according to the following expression.

(Amount of acetic acid in the aqueous zinc sulfate solution) = (Amount of acetic acid coming into the aqueous zinc sulfate solution) - (Amount of acetic acid coming out of the aqueous zinc sulfate solution)

In the expression, (Amount of acetic acid coming into the aqueous zinc sulfate solution) refers to the amount of acetic acid in a starting material monocyclic aromatic hydrocarbon such as benzene.

(Amount of acetic acid coming out of the aqueous zinc sulfate solution) refers to the amount of acetic acid in an oil phase (monocyclic aromatic hydrocarbon reaction solution) after reaction.

<Acetic acid concentration in monocyclic aromatic hydrocarbon and reaction solution of monocyclic aromatic hydrocarbon (oil phase after reaction)>

An acetic acid concentration in a monocyclic aromatic hydrocarbon (benzene) and a reaction solution of the monocyclic aromatic hydrocarbon was analyzed by measurement using a GC-2014 gas chromatograph apparatus manufactured by Shimadzu Corp.

When the acetic acid concentration in the monocyclic aromatic hydrocarbon or the reaction solution was 0.5 ppm or lower, sample analysis was carried out by measurement based on a calibration curve based on a distribution ratio obtained by adding water to a measurement sample and performing extraction and concentration.

(Ratio of acetic acid mass to mass of zirconia-containing ruthenium catalyst)

A ratio of an acetic acid mass to a zirconia-containing ruthenium catalyst mass was determined as a ratio of a total solid catalyst concentration (mg/L) in the reaction solution to the acetic acid concentration (mg/L) in the aqueous zinc sulfate solution.

Specifically, the reaction solution, the aqueous zinc sulfate solution, and the catalyst were extracted in a completely mixed state from the reaction system and left standing for separation. The acetic acid concentration in the aqueous zinc sulfate solution was measured using the analysis apparatus described above. The catalyst was determined in terms of a solid concentration in an aqueous sulfate solution by filtration and drying treatment, and the ratio of an acetic acid mass to a catalyst mass was thereby calculated.

(Zinc concentration in catalyst solid matter)

A zinc concentration in catalyst solid matter was measured using a ZSX Primus II fluorescent analysis apparatus manufactured by Rigaku Holdings Corp.

In the case of analyzing a small amount of a sample, the catalyst solid matter was dissolved in hydrochloric acid for toxic metal measurement, and a concentration of zinc dissolved in the solution was measured using an SPS3520UV-DD ICP apparatus manufactured by Hitachi High-Tech Corp.

(Average crystallite diameter of ruthenium)

An average crystallite diameter of ruthenium was determined according to the Scherrer equation from the extent of a diffraction peak at a diffraction angle ($2\theta$) of 44° of the ruthenium metal obtained using an XRD-6100 X-ray diffraction apparatus manufactured by Shimadzu Corp.

(Conversion rate of benzene and selection rate of cyclohexene)

A 1 L high-pressure autoclave reactor was charged with the catalyst and hydrogen. While a temperature and a hydrogen pressure were set, a predetermined amount of benzene was added thereto as a monocyclic aromatic hydrocarbon at once. Reaction based on a batch method was evaluated at the predetermined reaction temperature and hydrogen pressure.

The reaction based on the batch method, also called batch reaction, is a method that involves charging an autoclave with an aqueous zinc sulfate solution containing a catalyst, setting a predetermined temperature, pressure, and time, then adding a starting material benzene once at the start of reaction, and recovering the whole amount after the completion of reaction. After benzene addition, a portion of the reaction solution was extracted and analyzed using a gas chromatograph (GC-2014 manufactured by Shimadzu Corp.) equipped with an FID detector. A conversion rate of the reaction and a selection rate were collected over time according to the expressions (3) and (4) given below on the basis of a concentration analysis value from the experiment, and a selection rate at a conversion rate of 50% was further determined by analysis data plot.

Also, a 4 L continuous stirred tank reactor was charged with the catalyst, and benzene and hydrogen were continuously added thereto. Reaction was evaluated by a flow method of examining a reaction process. The reaction based on the flow method is a method that involves continuously adding a starting material benzene to a reactor and continuously recovering a reacted product from the reactor. This reaction can be continuously performed under constant conditions over a long period. A benzene conversion rate is adjusted by controlling a temperature, a pressure, and an amount of the catalyst as well as a benzene flow rate. In this respect, a selection rate of cyclohexene can be determined.

In the present Example, the benzene conversion rate was set to approximately 50%. As in the batch method, the reaction solution was analyzed using a gas chromatograph (GC-2014 manufactured by Shimadzu Corp.) equipped with an FID detector. The benzene conversion rate and the selection rate of cyclohexene were determined according to the expressions (3) and (4) given below on the basis of a concentration analysis value from the experiment.

Although the reaction performance of the continuous stirred tank reactor is theoretically lower than the selection rate of the reaction determined for the batch reactor, industrial production is performed in this continuous stirred tank reaction format because of its high production efficiency. Examples of the batch evaluation and Examples of the continuous flow method were summarized in Tables 3 and 4, respectively, because evaluation values differ therebetween.

$$\text{Conversion rate of benzene (\%)} = \frac{\text{The number of moles of benzene consumed through reaction}}{\text{The number of moles of benzene supplied to reaction}} \times 100 \qquad (3)$$

$$\text{Selection rate of cyclohexene (\%)} = \frac{\text{The number of moles of cyclohexene produced through reaction}}{\text{The number of moles of cyclohexane produced through reaction} + \text{The number of moles of cyclohexane produced through reaction}} \times 100 \qquad (4)$$

[Example 2-1]

(Preparation of catalyst)

20 g of ruthenium chloride hydrate (containing 40% (mass fraction) of ruthenium) and 8 g of zinc chloride were added with stirring to 500 mL of distilled water warmed to 80°C, and dissolved therein. 100 mL of a 5 N aqueous sodium hydroxide solution was added thereto, and stirring was further continued at 80°C for 2 hours to obtain a black precipitated solid. The solution containing the solid was cooled and filtered, and the recovered solid was added into 500 mL of a 1 N aqueous sodium hydroxide solution, followed by three repetitions of alkali washing and filtration operation. Then, water washing and filtration were further repeated five times using 500 mL of distilled water. Subsequently, the solid was added to a measuring cylinder containing an aqueous zinc sulfate solution provided in advance, and adjusted to a zinc sulfate concentration of 0.62 mol/L, and 500 mL of the resulting solution was added to a 1 L autoclave. Further, 45 g of a zirconia powder was added thereto. The 1 L autoclave was set and subjected to increase in temperature and pressure with stirring. Reduction treatment for 5 hours was carried out with 5 MPa of hydrogen having a temperature of 150°C.

Then, the 1 L autoclave was cooled, and the solution containing a ruthenium catalyst containing reduced zirconia was recovered into a beaker, which was then left standing, followed by operation of removing a supernatant by decantation. Further, distilled water was added thereto, and the beaker was stirred and left standing, followed by five repetitions of operation of exchanging a supernatant by decantation to collect 53.1 g (based on a dry mass) of a wet ruthenium catalyst containing zirconia.

The average crystallite diameter of ruthenium in the zirconia-containing ruthenium catalyst was 5.1 nm.

(Partial hydrogenation of benzene)

A 1 L autoclave was charged with 280 mL of a solution containing a catalyst slurry that contained 12 g (based on a dry mass) of the zirconia-containing ruthenium catalyst and had an acetic acid concentration adjusted to 5 mg/L and a zinc sulfate concentration adjusted to 0.62 mol/L.

The autoclave was purged with hydrogen with stirring and heated to 145°C. Then, hydrogen was further injected thereto, and the reaction pretreatment of a catalyst slurry was performed while the total pressure was kept at 5 MPa for 22 hours.

Subsequently, the pressure of the autoclave was temporarily lowered to 3 MPa. Then, 140 mL of benzene was injected thereto together with hydrogen and reacted with high-speed stirring at a total pressure of 5 MPa.

In a reaction process, a conversion rate of benzene and a selection rate of cyclohexene were determined by extracting the reaction solution from the autoclave over time and compositionally analyzing the liquid phase by gas chromatography. After reaction, the reaction product and the catalyst slurry were recovered from the autoclave, and an acetic acid concentration in the aqueous zinc sulfate solution, a ratio of an acetic acid mass to a mass of the added zirconia-containing ruthenium catalyst, and a concentration of zinc contained in the zirconia-containing ruthenium catalyst were measured.

Table 3 below shows each analysis value and the selection rate of cyclohexene at a benzene conversion rate of 50%.

[Example 2-2]

A 1 L autoclave was charged with 280 mL of a solution containing a catalyst slurry that contained 12 g (based on a dry mass) of the zirconia-containing ruthenium catalyst prepared in Example 2-1 and had an acetic acid concentration adjusted to 80 mg/L and a zinc sulfate concentration adjusted to 0.62 mol/L.

The autoclave was purged with hydrogen with stirring and heated to 145°C. Then, hydrogen was further injected thereto, and the reaction pretreatment of a catalyst slurry was performed while the total pressure was kept at 5 MPa for 22 hours.

Subsequently, the pressure of the autoclave was temporarily lowered to 3 MPa. Then, 140 mL of benzene was injected thereto together with hydrogen and reacted with high-speed stirring at a total pressure of 5 MPa.

A conversion rate of benzene and a selection rate of cyclohexene were determined from results of extracting the reaction solution over time and compositionally analyzing the liquid phase by gas chromatography.

After reaction, the reaction product and the catalyst slurry were recovered from the autoclave, and an acetic acid concentration in the aqueous zinc sulfate solution, a ratio of an acetic acid mass to a mass of the added catalyst, and a concentration of zinc contained in the zirconia-containing ruthenium catalyst were measured.

Table 3 below shows each analysis value and the selection rate of cyclohexene at a benzene conversion rate of 50%.

[Comparative Example 2-1]

A 1 L autoclave was charged with 280 mL of a solution containing a catalyst slurry that contained 12 g (based on a dry mass) of the zirconia-containing ruthenium catalyst prepared in Example 2-1 and had an acetic acid concentration adjusted to 120 mg/L and a zinc sulfate concentration adjusted to 0.62 mol/L.

The autoclave was purged with hydrogen with stirring and heated to 145°C. Then, hydrogen was further injected thereto, and the reaction pretreatment of a catalyst slurry was performed while the total pressure was kept at 5 MPa for 22 hours.

Subsequently, the pressure of the autoclave was temporarily lowered to 3 MPa. Then, 140 mL of benzene was injected thereto together with hydrogen and reacted with high-speed stirring at a total pressure of 5 MPa.

A conversion rate of benzene and a selection rate of cyclohexene were determined from results of extracting the reaction solution over time and compositionally analyzing the liquid phase by gas chromatography.

After reaction, the reaction product and the catalyst slurry were recovered from the autoclave, and an acetic acid concentration in the aqueous zinc sulfate solution, a ratio of an acetic acid mass to a mass of the added catalyst, and a concentration of zinc contained in the zirconia-containing ruthenium catalyst were measured.

Table 3 below shows each analysis value and the selection rate of cyclohexene at a benzene conversion rate of 50%.

[Comparative Example 2-2]

A 1 L autoclave was charged with 280 mL of a solution containing a catalyst slurry that contained 12 g (based on a dry mass) of the zirconia-containing ruthenium catalyst prepared in Example 2-1 and had an acetic acid concentration adjusted to 230 mg/L and a zinc sulfate concentration adjusted to 0.62 mol/L.

The autoclave was purged with hydrogen with stirring and heated to 145°C. Then, hydrogen was further injected thereto, and the reaction pretreatment of a catalyst slurry was performed while the total pressure was kept at 5 MPa for 22 hours.

Subsequently, the pressure of the autoclave was temporarily lowered to 3 MPa. Then, 140 mL of benzene was injected thereto together with hydrogen and reacted with high-speed stirring at a total pressure of 5 MPa.

A conversion rate of benzene and a selection rate of cyclohexene were determined from results of extracting the reaction solution over time and compositionally analyzing the liquid phase by gas chromatography.

After reaction, the reaction product and the catalyst slurry were recovered from the autoclave, and an acetic acid concentration in the aqueous zinc sulfate solution, a ratio of an acetic acid mass to a mass of the added catalyst, and a concentration of zinc contained in the zirconia-containing ruthenium catalyst were measured.

Table 3 below shows each analysis value and the selection rate of cyclohexene at a benzene conversion rate of 50%.

[Comparative Example 2-3]

The zirconia-containing ruthenium catalyst and the reaction solution reacted in Example 2-2 were recovered from the autoclave. An aromatic hydrocarbon component in the reaction solution was removed, and the catalyst slurry was further treated by nitrogen blow to the extent that the odor of aromatic hydrocarbon became imperceptible.

240 mL of the catalyst slurry was collected in a completely mixed state. 10 mL of a separately prepared aqueous solution having an acetic acid concentration adjusted to 2.01 g/L and a zinc sulfate concentration adjusted to 0.62 mol/L was added thereto, and a 1 L autoclave was charged with the mixture. The autoclave was purged with hydrogen with stirring and heated to 145°C. Then, hydrogen was further injected thereto, and the reaction pretreatment of a catalyst slurry was performed while the total pressure was kept at 5 MPa for 22 hours.

Subsequently, the pressure of the autoclave was temporarily lowered to 3 MPa. Then, 140 mL of benzene was injected thereto together with hydrogen and reacted with high-speed stirring at a total pressure of 5 MPa.

A conversion rate of benzene and a selection rate of cyclohexene were determined from results of extracting the reaction solution over time and compositionally analyzing the liquid phase by gas chromatography.

After reaction, the reaction product and the catalyst slurry were recovered from the autoclave, and an acetic acid concentration in the aqueous zinc sulfate solution, a ratio of an acetic acid mass to a mass of the added catalyst, and a concentration of zinc contained in the zirconia-containing ruthenium catalyst were measured.

Table 3 below shows each analysis value and the selection rate of cyclohexene at a benzene conversion rate of 50%.

[Example 2-3]

The zirconia-containing ruthenium catalyst and the reaction solution reacted in Comparative Example 2-2 were recovered from the autoclave. An aromatic hydrocarbon component in the reaction solution was removed, and the catalyst slurry was further treated by nitrogen blow to the extent that the odor of aromatic hydrocarbon became imperceptible.

240 mL of the catalyst slurry was collected in a completely mixed state and left standing to precipitate catalyst solid matter. Then, 140 mL of a supernatant was removed, and the residue was diluted to 280 mL by fresh addition of a 0.62 mol/L aqueous zinc sulfate solution.

Subsequently, the catalyst slurry was stirred for 5 minutes and left standing to precipitate catalyst solid matter. Then, 180 mL of a supernatant was removed, and the residue was diluted to 280 mL by fresh addition of a 0.62 mol/L aqueous zinc sulfate solution. This operation was repeated twice.

A 1 L autoclave was charged with 280 mL of the treated catalyst slurry, purged with hydrogen with stirring, and heated to 145°C. Then, hydrogen was further injected thereto, and the pretreatment of a catalyst was performed while the total pressure was kept at 5 MPa for 22 hours.

Subsequently, the pressure of the autoclave was temporarily lowered to 3 MPa. Then, 140 mL of benzene was injected thereto together with hydrogen and reacted with high-speed stirring at a total pressure of 5 MPa.

A conversion rate of benzene and a selection rate of cyclohexene were determined from results of extracting the reaction solution over time and compositionally analyzing the liquid phase by gas chromatography.

After reaction, the reaction product and the catalyst slurry were recovered from the autoclave, and an acetic acid concentration in the aqueous zinc sulfate solution, a ratio of an acetic acid mass to a mass of the added catalyst, and a concentration of zinc contained in the zirconia-containing ruthenium catalyst were measured.

Table 3 below shows each analysis value and the selection rate of cyclohexene at a benzene conversion rate of 50%.

[Table 3]

|  |  | Acetic acid concentration in aqueous zinc sulfate solution (mg/L) | Mass ratio of acetic acid to catalyst (times) | Cyclohexene selection rate (%) | Zn concentration in catalyst (% by mass) |
|---|---|---|---|---|---|
| Example 2-1 | Acetic acid was added to catalyst slurry before reaction | 5 | $1.2 \times 10^{-4}$ | 84.8 | 1.5 |
| Example 2-2 | Acetic acid was added to catalyst slurry before reaction | 80 | $1.9 \times 10^{-3}$ | 84.3 | 1.4 |
| Comparative Example 2-1 | Acetic acid was added to catalyst slurry before reaction | 120 | $2.8 \times 10^{-3}$ | 81.7 | 1.3 |
| Comparative Example 2-2 | Acetic acid was added to catalyst slurry before reaction | 230 | $5.4 \times 10^{-3}$ | 78.6 | 1.1 |
| Comparative Example 2-3 | Oil matter in reaction solution of Example 2-2 was removed, and additional acetic acid was added | 157 | $3.8 \times 10^{-3}$ | 81.9 | 1.2 |
| Example 2-3 | Oil matter in reaction solution of Comparative Example 2-2 was removed, and a portion of aqueous zinc sulfate solution was replaced | 10 | $2.7 \times 10^{-4}$ | 84.9 | 1.1 |

[Example 2-4]

A continuous stirred tank reactor was charged with 1200 mL of a solution that contained 50 g (based on a dry mass) of the zirconia-containing ruthenium catalyst prepared in the same manner as in Example 2-1 and had a zinc sulfate concentration of 0.62 mol/L.

This continuous stirred tank reactor has an oil-water separation tank as an attached tank and is provided with Teflon(R) coating on the inner surface.

Subsequently, benzene adjusted in advance so as to have 0.01 mg/L acetic acid was supplied at 1.2 L/hr to the continuous stirred tank reactor under hydrogen pressure of 5 MPa at 145°C to continuously perform the partial hydrogenation reaction of benzene.

In this operation, water was supplied such that a water phase containing the zirconia-containing ruthenium catalyst in the reaction system always had constant composition. A reaction product composed of benzene, cyclohexene, and cyclohexane was continuously recovered from the oil-water separation tank.

The volume ratio between the water phase and an oil phase in the reactor was constantly kept at water phase:oil phase = 2:1.

A conversion rate of benzene and a selection rate of cyclohexene were determined from results of collecting a reaction solution coming out of the oil-water separation tank after 22 hours from the start of reaction and compositionally analyzing the liquid phase by gas chromatography.

A catalyst slurry was collected following the sampling of the reaction solution from a catalyst sampling line established in the continuous stirred tank reactor, and an acetic acid concentration in the aqueous zinc sulfate solution, a ratio of an acetic acid mass to a mass of the catalyst in the reactor, and a concentration of zinc contained in the zirconia-containing ruthenium catalyst were measured.

Table 4 below shows each analysis value, the conversion rate of benzene, and the selection rate of cyclohexene.

[Example 2-5]

The reaction of Example 2-4 was continued. After 24 hours from the start of reaction, 600 mL of a catalyst slurry containing the reaction solution was extracted from the continuous stirred tank reactor, while the continuous stirred tank reactor was charged with 400 mL of the same catalyst slurry containing zinc sulfate as that initially added in Example 2-4.

An aromatic hydrocarbon component was removed from the extracted catalyst slurry containing the reaction solution, and the catalyst slurry was further treated by nitrogen blow to the extent that the odor of aromatic hydrocarbon became imperceptible.

Subsequently, nitrogen containing 7% by volume of oxygen was contacted therewith at 70°C for 1 hour. Then, the reactor was purged with nitrogen and further purged with hydrogen.

Then, the reactor was heated to 140°C and stirred for 4 hours while the internal pressure of the system was kept at 0.5 MPa in a hydrogen atmosphere. Operation of restoring catalytic activity was performed using the whole amount of the catalyst slurry.

In this way, operation of extracting 400 mL of the catalyst slurry from the reactor as mentioned above, restoring catalytic activity, and bringing the slurry thus treated back to the reactor was continued at a frequency of once per day while the reaction was continued.

A conversion rate of benzene and a selection rate of cyclohexene were determined from results of collecting a reaction solution coming out of the oil-water separation tank after 118 hours from the start of reaction and compositionally analyzing the liquid phase by gas chromatography.

A catalyst slurry was collected following the sampling of the reaction solution from a catalyst sampling line established in the continuous stirred tank reactor, and an acetic acid concentration in the aqueous zinc sulfate solution, a ratio of an acetic acid mass to a mass of the catalyst in the reactor, and a concentration of zinc contained in the zirconia-containing ruthenium catalyst were measured.

Table 4 below shows each analysis value, the conversion rate of benzene, and the selection rate of cyclohexene.

[Example 2-6]

A continuous stirred tank reactor was charged with 1200 mL of a solution that contained 50 g (based on a dry mass) of the zirconia-containing ruthenium catalyst prepared in the same manner as in Example 2-1 and had a zinc sulfate concentration of 0.62 mol/L.

Subsequently, benzene adjusted in advance so as to have 0.5 mg/L acetic acid was supplied at 1.2 L/hr to the continuous stirred tank reactor under hydrogen pressure of 5 MPa at 145°C to continuously perform the partial hydrogenation reaction of benzene.

In this operation, water was supplied such that a water phase containing the zirconia-containing ruthenium catalyst in the reaction system always had constant composition. A reaction product composed of benzene, cyclohexene, and cyclohexane was continuously recovered from the oil-water separation tank.

The volume ratio between the water phase and an oil phase in the reactor was constantly kept at water phase:oil phase = 2:1.

Reaction selectivity was determined from results of collecting a reaction solution coming out of the oil-water separation tank after 22 hours from the start of reaction and compositionally analyzing the liquid phase by gas chromatography.

A catalyst slurry was collected following the sampling of the reaction solution from a catalyst sampling line established in the continuous stirred tank reactor, and an acetic acid concentration in the aqueous zinc sulfate solution, a ratio of an acetic acid mass to a mass of the catalyst in the reactor, and a concentration of zinc contained in the zirconia-containing ruthenium catalyst were measured.

Table 4 below shows each analysis value, the conversion rate of benzene, and the selection rate of cyclohexene.

[Example 2-7]

The reaction of Example 2-6 was continued. After 24 hours from the start of reaction, 600 mL of a catalyst slurry containing the reaction solution was extracted from the continuous stirred tank reactor, while the continuous stirred tank reactor was charged with 400 mL of the same catalyst slurry containing zinc sulfate as that initially added in Example 2-4.

An aromatic hydrocarbon component was removed from the extracted catalyst slurry containing the reaction solution, and the catalyst slurry was further treated by nitrogen blow to the extent that the odor of aromatic hydrocarbon became imperceptible.

Subsequently, nitrogen containing 7% by volume of oxygen was contacted therewith at 70°C for 1 hour. Then, the reactor was purged with nitrogen and further purged with hydrogen.

Then, the reactor was heated to 140°C and stirred for 4 hours while the internal pressure of the system was kept at 0.5 MPa in a hydrogen atmosphere. Operation of restoring catalytic activity was performed using the whole amount of the catalyst slurry.

In this way, operation of extracting 400 mL of the catalyst slurry from the reactor as mentioned above, restoring catalytic activity, and bringing the slurry thus treated back to the reactor was continued at a frequency of once per day while the reaction was continued.

A conversion rate of benzene and a selection rate of cyclohexene were determined from results of collecting a reaction solution coming out of the oil-water separation tank after 118 hours from the start of reaction and compositionally analyzing the liquid phase by gas chromatography.

A catalyst slurry was collected following the sampling of the reaction solution from a catalyst sampling line established in the tank reactor, and an acetic acid concentration in the aqueous zinc sulfate solution, a ratio of an acetic acid mass to a mass of the catalyst in the reactor, and a concentration of zinc contained in the zirconia-containing ruthenium catalyst were measured.

Table 4 below shows each analysis value, the conversion rate of benzene, and the selection rate of cyclohexene.

[Example 2-8]

A continuous stirred tank reactor was charged with 1200 mL of a solution that contained 50 g (based on a dry mass) of the zirconia-containing ruthenium catalyst prepared in the same manner as in Example 2-1 and had a zinc sulfate concentration of 0.62 mol/L.

Subsequently, benzene adjusted in advance so as to have 1 mg/L acetic acid was supplied at 1.2 L/hr to the continuous stirred tank reactor under hydrogen pressure of 5 MPa at 145°C to continuously perform the partial hydrogenation reaction of benzene.

In this operation, water was supplied such that a water phase containing the zirconia-containing ruthenium catalyst in the reaction system always had constant composition. A reaction product composed of benzene, cyclohexene, and cyclohexane was continuously recovered from the oil-water separation tank.

The volume ratio between the water phase and an oil phase in the reactor was constantly kept at water phase:oil phase = 2:1.

A conversion rate of benzene and a selection rate of cyclohexene were determined from results of collecting a reaction solution coming out of the oil-water separation tank after 22 hours from the start of reaction and compositionally analyzing the liquid phase by gas chromatography.

A catalyst slurry was collected following the sampling of the reaction solution from a catalyst sampling line established in the continuous stirred tank reactor, and an acetic acid concentration in the aqueous zinc sulfate solution, a ratio of an acetic acid mass to a mass of the catalyst in the reactor, and a concentration of zinc contained in the zirconia-containing ruthenium catalyst were measured.

Table 4 below shows each analysis value, the conversion rate of benzene, and the selection rate of cyclohexene.

[Example 2-9]

The reaction of Example 2-8 was continued. After 24 hours from the start of reaction, 600 mL of a catalyst slurry containing the reaction solution was extracted from the continuous stirred tank reactor, while the continuous stirred tank reactor was charged with 400 mL of the same catalyst slurry containing zinc sulfate as that initially added in Example 2-8.

An aromatic hydrocarbon component was removed from the extracted catalyst slurry containing the reaction solution, and the catalyst slurry was further treated by nitrogen blow to the extent that the odor of aromatic hydrocarbon became imperceptible.

Subsequently, nitrogen containing 7% by volume of oxygen was contacted therewith at 50°C for 1 hour. Then, the reactor was purged with nitrogen and further purged with hydrogen.

Then, the reactor was heated to 140°C and stirred for 4 hours while the internal pressure of the system was kept at 0.5 MPa in a hydrogen atmosphere. Operation of restoring catalytic activity was performed using the whole amount of the catalyst slurry.

In this way, operation of extracting 400 mL of the catalyst slurry from the reactor as mentioned above, restoring catalytic activity, and bringing the slurry thus treated back to the reactor was continued at a frequency of once per day while the reaction was continued.

A conversion rate of benzene and a selection rate of cyclohexene were determined from results of collecting a reaction solution coming out of the oil-water separation tank after 118 hours from the start of reaction and compositionally analyzing the liquid phase by gas chromatography.

A catalyst slurry was collected following the sampling of the reaction solution from a catalyst sampling line established in the continuous stirred tank reactor, and an acetic acid concentration in the aqueous zinc sulfate solution, a ratio of an acetic acid mass to a mass of the catalyst in the reactor, and a concentration of zinc contained in the zirconia-containing ruthenium catalyst were measured.

Table 4 below shows each analysis value, the conversion rate of benzene, and the selection rate of cyclohexene.

[Example 2-10]

A continuous stirred tank reactor was charged with 1200 mL of a solution that contained 50 g (based on a dry mass) of the zirconia-containing ruthenium catalyst prepared in the same manner as in Example 2-1 and had a zinc sulfate concentration of 0.62 mol/L.

Subsequently, benzene adjusted in advance so as to have 4 mg/L acetic acid was supplied at 1.2 L/hr to the continuous stirred tank reactor under hydrogen pressure of 5 MPa at 145°C to continuously perform the partial hydrogenation reaction of benzene.

In this operation, water was supplied such that a water phase containing the zirconia-containing ruthenium catalyst in the reaction system always had constant composition. A reaction product composed of benzene, cyclohexene, and cyclohexane was continuously recovered from the oil-water separation tank.

The volume ratio between the water phase and an oil phase in the reactor was constantly kept at water phase:oil phase = 2:1.

After 20 hours from the start of reaction, 0.55 g of 96% by mass of sulfuric acid was added to the reactor through a supply line of replenished water in the reaction system. A conversion rate of benzene and a selection rate of cyclohexene were determined from results of collecting a reaction solution coming out of the oil-water separation tank after 22 hours from the start of reaction and compositionally analyzing the liquid phase by gas chromatography.

A catalyst slurry was collected following the sampling of the reaction solution from a catalyst sampling line established in the continuous stirred tank reactor, and an acetic acid concentration in the aqueous zinc sulfate solution, a ratio of an acetic acid mass to a mass of the catalyst in the reactor, and a concentration of zinc contained in the zirconia-containing ruthenium catalyst were measured.

Table 4 below shows each analysis value, the conversion rate of benzene, and the selection rate of cyclohexene.

[Comparative Example 2-4]

A continuous stirred tank reactor was charged with 1200 mL of a solution that contained 50 g (based on a dry mass) of the zirconia-containing ruthenium catalyst prepared in the same manner as in Example 2-1 and had a zinc sulfate concentration of 0.62 mg/L.

Subsequently, benzene adjusted in advance so as to have 6 mg/L acetic acid was supplied at 1.2 L/hr to the continuous stirred tank reactor under hydrogen pressure of 5 MPa at 145°C to continuously perform the partial hydrogenation reaction of benzene.

In this operation, water was supplied such that a water phase containing the zirconia-containing ruthenium catalyst in the reaction system always had constant composition. A reaction product composed of benzene, cyclohexene, and cyclohexane was continuously recovered from the oil-water separation tank.

The volume ratio between the water phase and an oil phase in the reactor was constantly kept at water phase:oil phase = 2:1.

A conversion rate of benzene and a selection rate of cyclohexene were determined from results of collecting a reaction solution coming out of the oil-water separation tank after 22 hours from the start of reaction and compositionally analyzing the liquid phase by gas chromatography.

A catalyst slurry was collected following the sampling of the reaction solution from a catalyst sampling line established in the continuous stirred tank reactor, and an acetic acid concentration in the aqueous zinc sulfate solution, a ratio of an acetic acid mass to a mass of the catalyst in the reactor, and a concentration of zinc contained in the zirconia-containing ruthenium catalyst were measured.

Table 4 below shows each analysis value, the conversion rate of benzene, and the selection rate of cyclohexene.

[Comparative Example 2-5]

The reaction of Comparative Example 2-4 was continued. After 24 hours from the start of reaction, 600 mL of a catalyst slurry containing the reaction solution was extracted from the continuous stirred tank reactor, while the continuous stirred tank reactor was charged with 400 mL of the same catalyst slurry containing zinc sulfate as that initially added in Example 2-8.

An aromatic hydrocarbon component was removed from the extracted catalyst slurry containing the reaction solution, and the catalyst slurry was further treated by nitrogen blow to the extent that the odor of aromatic hydrocarbon became imperceptible.

Subsequently, nitrogen containing 7% by volume of oxygen was contacted therewith at 50°C for 1 hour. Then, the reactor was purged with nitrogen and further purged with hydrogen.

Then, the reactor was heated to 140°C and stirred for 4 hours while the internal pressure of the system was kept at 0.5 MPa in a hydrogen atmosphere. Operation of restoring catalytic activity was performed using the whole amount of the catalyst slurry.

In this way, operation of extracting 400 mL of the catalyst slurry from the reactor as mentioned above, restoring catalytic activity, and bringing the slurry thus treated back to the reactor was continued at a frequency of once per day while the reaction was continued.

A conversion rate of benzene and a selection rate of cyclohexene were determined from results of collecting a reaction solution coming out of the oil-water separation tank after 118 hours from the start of reaction and compositionally analyzing the liquid phase by gas chromatography.

A catalyst slurry was collected following the sampling of the reaction solution from a catalyst sampling line established in the continuous stirred tank reactor, and an acetic acid concentration in the aqueous zinc sulfate solution, a ratio of an acetic acid mass to a mass of the catalyst in the reactor, and a concentration of zinc contained in the zirconia-containing ruthenium catalyst were measured.

Table 4 below shows each analysis value, the conversion rate of benzene, and the selection rate of cyclohexene.

[Comparative Example 2-6]

A continuous stirred tank reactor was charged with 1200 mL of a solution that contained 50 g (based on a dry mass) of the zirconia-containing ruthenium catalyst prepared in the same manner as in Example 2-1 and had a zinc sulfate concentration of 0.62 mg/L.

Subsequently, benzene adjusted in advance so as to have 10 mg/L acetic acid was supplied at 1.2 L/hr to the continuous stirred tank reactor under hydrogen pressure of 5 MPa at 145°C to continuously perform the partial hydrogenation reaction of benzene.

In this operation, water was supplied such that a water phase containing the zirconia-containing ruthenium catalyst in the reaction system always had constant composition. A reaction product composed of benzene, cyclohexene, and cyclohexane was continuously recovered from the oil-water separation tank.

The volume ratio between the water phase and an oil phase in the reactor was constantly kept at water phase:oil phase = 2:1.

A conversion rate of benzene and a selection rate of cyclohexene were determined from results of collecting a reaction solution coming out of the oil-water separation tank after 22 hours from the start of reaction and compositionally analyzing the liquid phase by gas chromatography.

A catalyst slurry was collected following the sampling of the reaction solution from a catalyst sampling line established in the continuous stirred tank reactor, and an acetic acid concentration in the aqueous zinc sulfate solution, a ratio of an acetic acid mass to a mass of the catalyst in the reactor, and a concentration of zinc contained in the zirconia-containing ruthenium catalyst were measured.

Table 4 below shows each analysis value, the conversion rate of benzene, and the selection rate of cyclohexene.

[Example 2-11]

The reaction of Comparative Example 2-6 was continued and shifted to benzene having an acetic acid concentration of 0.01 mg/L since 24 hours from the start of reaction. Further, operation of extracting 400 mL of a catalyst slurry containing the reaction solution, restoring catalytic activity, and bringing 400 mL of the slurry thus treated back to the reactor was continued at a frequency of once per day.

The catalyst slurry brought back to the reactor since 24 hours from the start of reaction was a solution extracted from the reactor, which was used after being treated until an acetic acid concentration was no longer detected. Specifically, the catalyst slurry was treated to the extent that the odor of an aromatic hydrocarbon component became imperceptible, followed by operation of contact with nitrogen containing 7% by volume of oxygen at 50°C for 1 hour, in the same manner as in Comparative Example 2-5. Subsequently, an acetic acid concentration was decreased by repetitions of operation of removing a supernatant by decantation at room temperature and adding a freshly prepared 0.62 mol/L aqueous zinc sulfate solution.

Then, 400 mL of the catalyst slurry treated by purging with nitrogen and hydrogen was heated to 140°C, and the reactor was heated to 140°C and stirred for 4 hours while the internal pressure of the system was kept at 0.5 MPa in a hydrogen atmosphere. The catalyst slurry treated by the restoration operation was secured and used at the time of catalyst addition.

A conversion rate of benzene and a selection rate of cyclohexene were determined from results of collecting a reaction solution coming out of the oil-water separation tank after 118 hours from the start of reaction and compositionally analyzing the liquid phase by gas chromatography.

A catalyst slurry was collected following the sampling of the reaction solution from a catalyst sampling line established in the continuous stirred tank reactor, and an acetic acid concentration in the aqueous zinc sulfate solution, a ratio of an acetic acid mass to a mass of the catalyst in the reactor, and a concentration of zinc contained in the zirconia-containing ruthenium catalyst were measured.

Table 4 below shows each analysis value, the conversion rate of benzene, and the selection rate of cyclohexene.

[Table 4]

| | | Acetic acid concentration in aqueous zinc sulfate solution (mg/L) | Acetic acid concentration in starting material benzene (mg/L) | Mass ratio of acetic acid to catalyst (times) | Reaction time (Hr) | Benzene conversion rate (%) | Cyclohexene selection rate (%) | Zn concentration in catalyst (% by mass) |
|---|---|---|---|---|---|---|---|---|
| Example 2-4 | Acetic acid was added to starting material benzene | 0.20 | 0.01 | $4 8\times10^{-6}$ | 22 | 50.9 | 75.3 | 1.5 |
| Example 2-5 | Reaction time of Example 2-4 was extended | 0.85 | 0.01 | $15\times10^{-5}$ | 118 | 51.4 | 75.8 | 1.3 |
| Example 2-6 | Acetic acid was added to starting material benzene | 10 | 0.5 | $2.4\times10^{-4}$ | 22 | 51.2 | 74.8 | 1.4 |
| Example 2-7 | Reaction time of Example 2-6 was extended | 43 | 0.5 | $7.7\times10^{-4}$ | 118 | 51.8 | 75.5 | 1.1 |
| Example 2-8 | Acetic acid was added to starting material benzene | 20 | 1 | $4.8\times10^{-4}$ | 22 | 50.5 | 75.0 | 1.2 |
| Example 2-9 | Reaction time of Example 2-8 was extended | 86 | 1 | $1.6\times10^{-3}$ | 118 | 51.1 | 75.1 | 1.1 |
| Example 2-10 | Acetic acid was added to starting material benzene | 82 | 4.0 | $2.0\times10^{-3}$ | 22 | 52.7 | 74.2 | 0.7 |
| Comparative Example 2-4 | Acetic acid was added to starting material benzene | 123 | 6.0 | $3.0\times10^{-3}$ | 22 | 52.3 | 71.3 | 0.9 |
| Comparative Example 2-5 | Reaction time of Comparative Example 2-4 was extended | 521 | 6.0 | $9.4\times10^{-3}$ | 118 | 52.8 | 68.6 | 0.8 |
| Comparative Example 2-6 | Acetic acid was added to starting material benzene | 206 | 10.0 | $4.9\times10^{-3}$ | 22 | 51.6 | 69.3 | 0.9 |
| Example 2-11 | Oil matter in reaction solution of Comparative Example 2-6 was removed, and a portion of aqueous zinc sulfate solution was replaced | 45 | 0.01 | $81\times10^{-4}$ | 118 | 52.1 | 74.9 | 0.8 |

[Example 3-1]

In the present Example, an extraction step of extracting and separating cyclohexene and cyclohexane obtained through reaction and unreacted benzene using dimethylacetamide as an extracting agent was carried out after the partial hydrogenation reaction of benzene. Further, benzene was recovered from the mixed solution of the extracting agent and the unreacted benzene and washed with water, and the resulting benzene was brought back to a reactor. In this step, dimethylamine and acetic acid generated as dimethylacetamide degradants in the extraction step were reduced by the water washing of benzene as mentioned above, and operation of circulating and recycling the benzene to partial hydrogenation reaction was performed.

In order to acceleratingly reproduce the state of the bottom of a benzene recovery column for recovering dimethylacetamide and unreacted benzene, a 1 L autoclave was charged with 600 g of dimethylacetamide containing 5.027 g of water and 1.795 g of acetic acid and subjected to heat treatment at 200°C for 42 hours.

400 g of the treated solution was collected, and 1 kg of benzene was added thereto. Distillation under reduced pressure was carried out under conditions of 80°C and 80 kPa. After the distillation under reduced pressure was performed for 3 hours, 170 g of an initial distillate was recovered. The recovered solution was adjusted to 35 kg by the addition of benzene to prepare a benzene-adjusted solution corresponding to the top of the benzene recovery column.

Components of the benzene-adjusted solution were analyzed and were consequently 46.9 mg/L dimethylamine and 8.31 mg/L acetic acid.

The benzene-adjusted solution was subjected to water washing treatment with a solution temperature controlled to a constant temperature of 40°C, using a water washing experimental apparatus where a 500 mm packed bed of a Pall ring of 5/8 inches in outside diameter was established in the central part of the apparatus having a piping outside diameter of 150 mm and a height of 1000 mm.

Water was added downward at a total of 11.0 L/hr from four nozzles established in the upper part of the water washing experimental apparatus. On the other hand, the benzene-adjusted solution was also added upward at 20.7 L/hr from four nozzles established in the lower part of the apparatus so that benzene was contacted with 0.53 times its volume of water. Then, water for water washing was extracted from the lower part of the water washing experimental apparatus while 32.0 kg of benzene washed with water was recovered from the upper part of the water washing experimental apparatus.

Components of the benzene thus washed with water were analyzed and were consequently 6.45 mg/L dimethylamine and 0.82 mg/L acetic acid.

Subsequently, the partial hydrogenation reaction of benzene was continuously performed in the same manner as in Example 1-6 except that benzene washed with water as mentioned above was used.

After 20 hours from the start of reaction, 0.2 g of 96% by mass of sulfuric acid was added to the reactor through a supply line of replenished water in the reaction system. A benzene conversion rate of 50.6% and a cyclohexene selection rate of 75.4% were obtained as a result of collecting a reaction solution coming out of the oil-water separation tank after 22 hours from the start of reaction and compositionally analyzing the liquid phase by gas chromatography.

A catalyst slurry was collected following the sampling of the reaction solution from a catalyst sampling line established in the continuous stirred tank reactor. It was confirmed that: the aqueous zinc sulfate solution had a dimethylamine concentration of 137 mg/L, a nitrogen concentration of 51 mg/L, and an acetic acid concentration of 17 mg/L; in the reactor, a ratio of a dimethylamine mass to a mass of the catalyst was $3.2 \times 10^{-3}$ times, a ratio of a nitrogen mass to the mass of the catalyst was $1.2 \times 10^{-3}$ times, and a ratio of an acetic acid mass to the mass of the catalyst was $4.2 \times 10^{-4}$ times; and a concentration of zinc contained in the zirconia-containing ruthenium catalyst was measured and was consequently 1.26% by mass.

The present application is based on Japanese Patent Application Nos. 2022-125754 and 2022-125785 filed in the Japan Patent Office on August 5, 2022, the contents of which are incorporated herein by reference in their entirety.

**Industrial Applicability**

The present invention has industrial applicability as a method for producing a cycloolefin which efficiently produces cycloolefin at a high selection rate over a long time while suppressing reduction in selection rate of cycloolefin.

**Claims**

**1.** A method for producing a cycloolefin, comprising subjecting a monocyclic aromatic hydrocarbon to partial hydrogenation reaction with hydrogen in an aqueous zinc sulfate solution in the presence of a ruthenium catalyst, wherein

the aqueous zinc sulfate solution contains dimethylamine, and
the aqueous zinc sulfate solution has a nitrogen concentration of 0.5 to 3000 mg/L.

2. The method for producing the cycloolefin according to claim 1, wherein
the partial hydrogenation reaction of the monocyclic aromatic hydrocarbon is performed with the nitrogen concentration in the aqueous zinc sulfate solution containing the ruthenium catalyst adjusted to 0.5 to 3000 mg/L by replacing a portion of the aqueous zinc sulfate solution with a fresh aqueous zinc sulfate solution.

3. The method for producing the cycloolefin according to claim 1 or 2, wherein
a concentration of nitrogen contained in the monocyclic aromatic hydrocarbon is 0.003 to 35 mg/L.

4. The method for producing the cycloolefin according to claim 1 or 2, wherein
a ratio of a nitrogen mass to a mass of the ruthenium catalyst in the aqueous zinc sulfate solution is $5 \times 10^{-6}$ to $8 \times 10^{-2}$ times.

5. The method for producing the cycloolefin according to claim 1 or 2, wherein

the partial hydrogenation reaction is performed with
the nitrogen concentration in the aqueous zinc sulfate solution adjusted to 0.5 to 1500 mg/L,
a nitrogen concentration in the monocyclic aromatic hydrocarbon adjusted to 0.01 to 16 mg/L, and
a ratio of a nitrogen mass to a mass of the ruthenium catalyst in the aqueous zinc sulfate solution adjusted to $1 \times 10^{-5}$ to $5 \times 10^{-2}$ times.

6. The method for producing the cycloolefin according to claim 1 or 2, wherein

the aqueous zinc sulfate solution has a dimethylamine concentration of 1.7 to 9900 mg/L,
the monocyclic aromatic hydrocarbon has a dimethylamine concentration of 0.01 to 115 mg/L, and
a ratio of a dimethylamine mass to a mass of the ruthenium catalyst in the aqueous zinc sulfate solution is $2 \times 10^{-5}$ to 0.26 times.

7. The method for producing the cycloolefin according to claim 1 or 2, comprising:

a reaction step of subjecting the monocyclic aromatic hydrocarbon to partial hydrogenation reaction with hydrogen in the aqueous zinc sulfate solution;
an extraction step of extracting an unreacted form of the monocyclic aromatic hydrocarbon with a solvent containing a nitrogen-containing compound after the reaction step;
a water washing step of contacting the monocyclic aromatic hydrocarbon thus extracted with 0.05 to 1 times its volume of water; and
a circulation step of subjecting the monocyclic aromatic hydrocarbon thus washed with water to the reaction step, wherein
in the reaction step, a content of dimethylamine in the aqueous zinc sulfate solution is controlled to 40 to 1700 mg/L, and a ratio of a dimethylamine mass to a mass of the ruthenium catalyst is controlled to $8 \times 10^{-4}$ to $6 \times 10^{-2}$ times.

8. A method for producing a cycloolefin, comprising subjecting a monocyclic aromatic hydrocarbon to partial hydrogenation reaction with hydrogen in an aqueous zinc sulfate solution in the presence of a ruthenium catalyst, wherein the partial hydrogenation reaction is performed with an acetic acid concentration in the aqueous zinc sulfate solution adjusted to $1 \times 10^{-3}$ to 100 mg/L.

9. The method for producing the cycloolefin according to claim 8, wherein
the partial hydrogenation reaction of the monocyclic aromatic hydrocarbon is performed with the acetic acid concentration in the aqueous zinc sulfate solution containing the ruthenium catalyst adjusted to $1 \times 10^{-3}$ to 100 mg/L by replacing a portion of the aqueous zinc sulfate solution with a fresh aqueous zinc sulfate solution.

10. The method for producing the cycloolefin according to claim 8 or 9, wherein
a concentration of acetic acid contained in the monocyclic aromatic hydrocarbon is $1 \times 10^{-4}$ to 5 mg/L.

11. The method for producing the cycloolefin according to claim 8 or 9, wherein

a ratio of an acetic acid mass to a mass of the ruthenium catalyst in the aqueous zinc sulfate solution is in the range of $3 \times 10^{-9}$ to $2.5 \times 10^{-3}$ times.

12. The method for producing the cycloolefin according to claim 8 or 9, comprising:

a reaction step of subjecting the monocyclic aromatic hydrocarbon to partial hydrogenation reaction with hydrogen in the aqueous zinc sulfate solution;

an extraction step of extracting an unreacted form of the monocyclic aromatic hydrocarbon with a solvent containing a nitrogen-containing compound after the reaction step;

a water washing step of contacting the monocyclic aromatic hydrocarbon thus extracted with 0.05 to 1 times its volume of water; and

a circulation step of subjecting the monocyclic aromatic hydrocarbon thus washed with water to the reaction step, wherein

in the reaction step, a ratio of an acetic acid mass to a mass of the ruthenium catalyst in the aqueous zinc sulfate solution is controlled to $1 \times 10^{-6}$ to $5 \times 10^{-4}$ times.

13. The method for producing the cycloolefin according to claim 1 or 8, wherein

the ruthenium catalyst is a zirconia-containing ruthenium catalyst,

the method comprising the step of regenerating a portion or the whole of the zirconia-containing ruthenium catalyst, wherein

the zirconia-containing ruthenium catalyst thus regenerated is recycled.

14. The method for producing the cycloolefin according to claim 1 or 8, wherein

the ruthenium catalyst is a zirconia-containing ruthenium catalyst, and

the zirconia-containing ruthenium catalyst has a zinc concentration of 0.5 to 3.5% by mass.

15. The method for producing the cycloolefin according to claim 1 or 8, wherein

the monocyclic aromatic hydrocarbon comprises

any member selected from the group consisting of benzene, toluene, and benzene substituted by an alkyl group having 1 to 4 carbon atoms.

16. The method for producing the cycloolefin according to claim 1, wherein
the aqueous zinc sulfate solution has an acetic acid concentration of $1 \times 10^{-3}$ mg/L to 100 mg/L.

## INTERNATIONAL SEARCH REPORT

| International application No. |
| --- |
| **PCT/JP2023/027336** |

| A. | CLASSIFICATION OF SUBJECT MATTER |
| --- | --- |

***C07C 5/11***(2006.01)i; ***C07B 61/00***(2006.01)i; ***C07C 13/20***(2006.01)i
FI:  C07C5/11; C07C13/20; C07B61/00 300

According to International Patent Classification (IPC) or to both national classification and IPC

| B. | FIELDS SEARCHED |
| --- | --- |

Minimum documentation searched (classification system followed by classification symbols)

C07C5/11; C07B61/00; C07C13/20

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY (STN)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 5398082 B2 (ASAHI KASEI CHEMICALS CORPORATION) 29 January 2014 (2014-01-29) entire text | 1-16 |
| A | JP 63-152333 A (ASAHI CHEM IND CO LTD) 24 June 1988 (1988-06-24) entire text | 1-16 |
| A | JP 11-228457 A (MITSUBISHI CHEMICAL CORP) 24 August 1999 (1999-08-24) entire text | 1-16 |
| A | JP 7-267882 A (COSMO SOGO KENKYUSHO KK) 17 October 1995 (1995-10-17) entire text | 1-16 |
| A | CN 105130734 A (ZHENGZHOU UNIVERSITY) 09 December 2015 (2015-12-09) entire text | 1-16 |
| A | WO 2014/057417 A1 (VERSALIS S.P.A.) 17 April 2014 (2014-04-17) entire text | 1-16 |

☐ Further documents are listed in the continuation of Box C.  ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **06 September 2023** | **26 September 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2023/027336**

| Patent document cited in search report | | | Publication date (day/month/year) | Patent family member(s) | | | Publication date (day/month/year) |
|---|---|---|---|---|---|---|---|
| JP | 5398082 | B2 | 29 January 2014 | US 2015/0238925 A1 entire text<br>WO 2010/073481 A1<br>EP 2368634 A1<br>KR 10-2011-0054049 A<br>CN 102264471 A | | | |
| JP | 63-152333 | A | 24 June 1988 | JP 63-159329 A | | | |
| JP | 11-228457 | A | 24 August 1999 | (Family: none) | | | |
| JP | 7-267882 | A | 17 October 1995 | (Family: none) | | | |
| CN | 105130734 | A | 09 December 2015 | (Family: none) | | | |
| WO | 2014/057417 | A1 | 17 April 2014 | EP 2906345 A1 entire text<br>KR 10-2015-0068431 A<br>CN 104812487 A | | | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2115581 A
- JP 2138012 A
- JP 4777891 B
- JP 1849859 A
- JP 2892233 B
- JP 5254338 B
- JP 5147053 B
- JP 3125913 B

- JP 2634828 B
- JP 2886563 B
- JP 3841226 B
- JP 4397468 B
- JP 4033980 B
- JP 4025407 B
- JP 2022125754 A
- JP 2022125785 A

**Non-patent literature cited in the description**

- *IUPAC Nomenclature of Inorganic Chemistry*, 1989